(19) Europäisches Patentamt / European Patent Office / Office européen des brevets

(11) EP 2 214 665 B1

(12) # FASCICULE DE BREVET EUROPEEN

(45) Date de publication et mention de la délivrance du brevet:
**11.07.2018 Bulletin 2018/28**

(21) Numéro de dépôt: **08871540.4**

(22) Date de dépôt: **31.10.2008**

(51) Int Cl.:
*A61K 31/7072 (2006.01)* *A61K 31/47 (2006.01)*
*A61P 31/12 (2006.01)* *A61K 45/06 (2006.01)*
*A61K 38/05 (2006.01)* *A61K 38/00 (2006.01)*

(86) Numéro de dépôt international:
**PCT/FR2008/001538**

(87) Numéro de publication internationale:
**WO 2009/092897 (30.07.2009 Gazette 2009/31)**

(54) **Composé Q-VD-OPh pour utilisation dans le traitement d'une infection virale**

Q-VD-OPh zur Behandlung einer Virusinfektion

Compound Q-VD-OPh for use in the treatment of a viral infection

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **02.11.2007 FR 0707722**

(43) Date de publication de la demande:
**11.08.2010 Bulletin 2010/32**

(73) Titulaires:
- **Institut Pasteur 75724 Paris Cédex 15 (FR)**
- **Centre National de la Recherche Scientifique 75794 Paris Cedex 16 (FR)**
- **INSERM (Institut National de la Santé et de la Recherche Médicale) 75654 Paris Cedex 13 (FR)**
- **Université Paris-Sud 91400 Orsay (FR)**

(72) Inventeurs:
- **ESTAQUIER, Jérôme 95160 Montmorency (FR)**
- **LAFORGE, Mireille 94700 Maisons-ALFORT (FR)**
- **SENIK, Anna 75003 Paris (FR)**

(74) Mandataire: **Lavoix 2, place d'Estienne d'Orves 75441 Paris Cedex 09 (FR)**

(56) Documents cités:
**WO-A-02/18341 WO-A-99/18781**
**WO-A-2004/002961 WO-A-2004/058718**
**WO-A-2007/084730 US-A- 5 877 197**

- **ULRIKE MARTIN ET AL: "Antiviral effects of pan-caspase inhibitors on the replication of coxsackievirus B3" APOPTOSIS ; AN INTERNATIONAL JOURNAL ON PROGRAMMED CELL DEATH, KLUWER ACADEMIC PUBLISHERS, BO, vol. 12, no. 3, 9 janvier 2007 (2007-01-09), pages 525-533, XP019477438 ISSN: 1573-675X**
- **R. L. DEBIASI ET AL: "Caspase Inhibition Protects against Reovirus-Induced Myocardial Injury In Vitro and In Vivo" JOURNAL OF VIROLOGY, vol. 78, no. 20, octobre 2004 (2004-10), pages 11040-11050, XP002479654**
- **T. M. CASERTA, A. N. SMITH, A. D. GULTICE, M. A. REEDY, T. L. BROWN: "Q-VD-OPh, a broad spectrum caspase inhibitor with potent antiapoptotic properties" APOPTOSIS, vol. 8, 2003, pages 345-352, XP002479655**
- **KLEINSCHMIDT MALTE C ET AL: "Inhibition of apoptosis prevents West Nile virus induced cell death" BMC MICROBIOLOGY, BIOMED CENTRAL, LONDON, GB, vol. 7, no. 1, 29 mai 2007 (2007-05-29), page 49, XP021028169 ISSN: 1471-2180**
- **CHIOU S-K ET AL: "Inhibition of ICE-like Proteases Inhibits Apoptosis and Increases Virus Production during Adenovirus Infection" VIROLOGY, ACADEMIC PRESS,ORLANDO, US, vol. 244, no. 1, 25 avril 1998 (1998-04-25), pages 108-118, XP004845019 ISSN: 0042-6822**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 214 665 B1

## Description

L'invention relève du domaine des infections par le virus de l'Immunodéficience humaine (VIH).

L'invention concerne le composé Q-VD-OPh (N-(2(quinolyl)valyl-aspartyl-(2,6-difluorophénoxy)méthyl cétone), pour une utilisation pour la prophylaxie et/ou le traitement d'une infection virale provoquée par un virus de l'immunodéficience humaine (VIH).

L'invention a également pour objet l'utilisation du composé Q-VD-OPh, pour la préparation d'une composition pharmaceutique destinée à la prophylaxie et/ou au traitement d'une infection virale provoquée par un virus de l'immunodéficience humaine (VIH).

La présente invention concerne également une nouvelle composition antivirale et une nouvelle combinaison (ou kit) de composés appropriés pour une utilisation pour la prophylaxie et/ou le traitement d'une infection virale provoquée par un virus de l'immunodéficience humaine (VIH), qui comprennent, consistent essentiellement en ou consistent en :

(i) au moins le composé Q-VD-OPh et
(ii) au moins un autre agent antiviral, par exemple un inhibiteur de protéase virale ou un inhibiteur de transcriptase, notamment de transcriptase inverse.

La réplication virale est le processus par lequel un virus (à ADN ou à ARN) détourne et utilise la machine de la cellule qu'il infecte pour se multiplier. A titre d'exemple, les principales étapes de la réplication des rétrovirus et, en particulier, des virus VIH, sont les suivantes : (1) la fixation du virus à la surface d'une cellule d'un organisme animal ou humain par reconnaissance entre des protéines de surface du virus et des récepteurs à la surface de ladite cellule (par exemple le récepteur CD4) ; (2) la pénétration du virus dans le cytoplasme de la cellule par fusion de l'enveloppe du virus avec la membrane de la cellule ; (3) la décapsidation du virus (le virus se sépare de la matrice et de la capside, ce qui libère les deux copies du génome viral) ; (4) la transcription inverse des ARN viraux sous la forme d'un ADN proviral grâce à la transcriptase inverse (enzyme virale); (5) la migration de l'ADN proviral dans le noyau et l'intégration de cet ADN dans l'ADN de la cellule hôte sous l'effet de l'intégrase (enzyme virale) ; (6) la transcription de l'ADN de la cellule en ARN génomique (ARN messager (ARNm) non épissé) sous l'effet de l'ARN polymérase de la cellule ; (7) l'épissage de l'ARNm, par excision des introns, pour ne laisser que les exons (qui codent pour les protéines Gag, Pol et Env) ; (8) la traduction, dans le réticulum endoplasmique rugueux, de l'ARNm sous la forme de polypeptides; (9) la maturation des polypeptides dans l'appareil de Golgi, permettant d'obtenir des polypeptides fonctionnels ; (10) l'assemblage des particules virales à la surface de la membrane par l'accumulation des polyprotéines de structure multimérisées (Gag, p55)), des protéines virales non structurales (transcriptase inverse, intégrase, protéase) et des ARN viraux; (11) la libération des virions par bourgeonnement à la surface de la cellule infectée ; et enfin, (12) la maturation des virus.

Les virus ont développé diverses stratégies pour échapper au système immunitaire et faciliter leur dissémination au cours de l'infection. En particulier, le virus VIH a la particularité de provoquer l'effondrement complet du système immunitaire en s'attaquant à une cellule clé du système immunitaire, le lymphocyte T auxiliaire (lymphocyte T CD4+), qui exprime à sa surface la molécule CD4, récepteur spécifique du VIH. Les monocytes-macrophages, les cellules dendritiques, les cellules de Langerhans et les cellules microgliales cérébrales sont également des cibles du VIH. La disparition progressive des lymphocytes conduit à un défaut de contrôle de la réplication virale par le système immunitaire, à la destruction des organes lymphoïdes où a lieu la réponse immunitaire, et à l'installation du syndrome d'immunodéficience acquise (SIDA), avec apparition d'infections opportunistes sévères.

Les mécanismes responsables de la disparition des lymphocytes T CD4+ au cours de l'infection par le VIH sont complexes, et ils ne sont que partiellement élucidés.

La particule virale du VIH est composée d'une nucléocapside qui renferme le dimère d'ARN simple brin de polarité positive associé à la protéine de nucléocapside, des ARNt-Lysine et les enzymes virales (transcriptase inverse, protéase et intégrase). La nucléocapside est enfermée dans une gaine de protéines de matrice qui est recouverte d'une membrane lipidique empruntée à la cellule hôte au cours du bourgeonnement de la particule virale. Cette membrane est hérissée de spicules constituées d'oligomères de glycoprotéines d'enveloppe. L'étape de conversion de l'ARN en ADN bicaténaire au cours du cycle viral sous l'action d'une enzyme virale, la transcriptase inverse, est la principale caractéristique des rétrovirus.

Les gènes viraux gag, pol et env sont conservés chez l'ensemble des rétrovirus. Tous les produits qui en sont issus sont présents dans la particule virale. Ils proviennent du clivage de polyprotéines précurseurs. Les gènes gag et env codent pour des protéines de structure et le gène pol code pour plusieurs protéines enzymatiques.

Les protéines Gag sont issues du clivage de la polyprotéine Pr55gag par la protéase virale. Ce clivage libère la protéine de matrice, la protéine de capside, la protéine de nucléocapside, ainsi qu'une protéine de 6 kDa. De récents travaux suggèrent le rôle essentiel de ces processus dans la genèse de particules virales infectieuses (Sticht et al., 2005 ; Ternois et al., 2005). Ces récents travaux ont permis de générer, pour la première fois, un inhibiteur de l'assemblage du VIH.

Le précurseur d'enveloppe gp160 est clivé en une glycoprotéine de surface gp120 (gp130 pour le SIVmac) et en une protéine transmembranaire gp41 issue de la région C-terminale du précurseur. Au cours de sa maturation, le précurseur gp160 est glycosylé puis clivé par une protéase cellulaire au niveau de l'appareil de Golgi, puis exporté à la membrane plasmique. Les deux glycoprotéines issues du clivage demeurent associées par des liaisons non covalentes. Elles forment des hétéromères de glycoprotéines d'enveloppe qui s'associent en oligomères pour former les spicules du virion.

Le gène pol code pour trois protéines enzymatiques : la protéase, la transcriptase inverse et l'intégrase. Elles sont issues du clivage de la polyprotéine Gag-Pol (Pr160 gag-pol) au cours de la morphogenèse du virion. La dimérisation dans la cellule de la polyprotéine Gag-Pol révèle l'activité protéase codée par la région 5' de pol. La forme mature de la protéase, libérée par clivage autocatalytique reste sous forme dimérique p11/p11 et peut alors cliver d'autres sites présents sur les polyprotéines Pr160gag-pol et Pr55gag.

La transcriptase inverse est issue du clivage en deux étapes de la polyprotéine Pr160gag-pol par la protéase virale au cours de l'assemblage de la particule virale.

Située en position C-terminale de la région Pol de la polyprotéine Gag-Pol, l'intégrase est libérée sous forme d'une protéine de 32 kDa sous l'action de la protéase virale. Son oligomérisation est requise tant pour son incorporation dans la particule virale que pour exercer son activité d'intégration de l'ADN viral double brins linéaire dans le génome cellulaire.

L'ensemble de ces travaux souligne le rôle majeur de ou des protéases dans la genèse d'une particule virale infectieuse. Ainsi, outre l'utilisation d'inhibiteurs de la rétrotranscriptase ou d'analogues nucléosidiques, la thérapie anti-VIH dite thérapie antirétrovirale hautement active ou « HAART » ("Highly Active Anti-retroviral therapy") comporte aujourd'hui un ou plusieurs inhibiteurs de la protéase du VIH. Cette thérapie conduit à une inhibition de la réplication virale, une augmentation du nombre de lymphocytes T CD4 et à une amélioration clinique indiscutable.

Cependant, dans la mesure où aucun traitement actuel ne permet de guérir les malades du SIDA et où des isolats des virus VIH sont ou deviennent résistants aux traitements existants, il est nécessaire de trouver d'autres molécules antivirales permettant de lutter plus efficacement contre les infections virales en général et contre les infections par les rétrovirus tels que le VIH en particulier.

De nombreuses infections virales coïncident avec des dérèglements de mécanismes contrôlant la mort cellulaire (Barber, 2001). En particulier, de nombreux travaux indiquent une relation entre l'infection *in vitro* ou *in vivo* par le VIH et l'augmentation de la susceptibilité à l'apoptose des lymphocytes T. L'apoptose (ou mort cellulaire programmée ou encore, suicide cellulaire) est le processus par lequel des cellules déclenchent leur auto destruction en réponse à un signal (signal pro-apoptotique). L'environnement, les interactions entre cellules, l'absence de nourriture pour la cellule, l'infection par un agent pathogène sont quelques exemples de signal pro-apoptotique. L'apoptose est une forme morphologiquement et biochimiquement définie de mort cellulaire caractérisée *in vivo* par l'absence de réponse inflammatoire, l'activation de caspases et le clivage de nombreuses protéines, la fragmentation de l'ADN, la condensation de la chromatine, la contraction cellulaire et le désassemblage des structures cellulaires pour former des vésicules incorporées à la membrane (corps apoptotiques). *In vivo,* ce processus culmine avec la phagocytose de corps apoptotiques par d'autres cellules.

L'apoptose précoce d'une cellule infectée par un virus peut constituer un mécanisme de défense de l'hôte ; elle permet de limiter le nombre de particules virales libérées en interrompant la réplication virale. Les endonucléases cellulaires produites au cours de l'apoptose peuvent agir sur l'ADN viral et inhiber la synthèse des protéines virales, structurales et régulatrices et la formation de particules virales infectieuses limitant ainsi la dissémination des virions chez l'hôte.

Ainsi, de nombreux virus agissent sur la régulation des signaux intracellulaires apoptotiques soit pour se maintenir en vie, soit pour maintenir la cellule infectée viable, soit pour empêcher la cellule d'être agressée par les cellules effectrices du système immunitaire, et ainsi augmenter l'efficacité de la réplication virale et permettre une production plus importante de virions. La plupart des virus possèdent un ou plusieurs gènes permettant la synthèse de protéines dont l'effet est de réprimer, à différents stades, l'apoptose des cellules qu'ils infectent (protéines anti-apoptotiques). En retardant ou inhibant la mort de la cellule hôte, les virus favorisent la survie de la cellule qu'ils infectent et donc leur propre survie, jusqu'à favoriser l'apparition de cancers pour certains.

D'autres virus ont également développé des stratégies pour, au contraire, provoquer la mort des cellules qu'ils infectent, entraînant des déficits cellulaires, en particulier des déficits immunitaires (tels que ceux associés au sida), des déficits neuronaux (tels que ceux associés à la rage) et des déficits épithéliaux (tels que ceux associés aux fièvres hémorragiques). Dans le cas des déficits immunitaires seulement, les virus peuvent alors se propager. Certains virus sont en outre capables d'induire l'apoptose à une phase tardive de l'infection, ce qui permet la propagation des virions dans les cellules voisines tout en échappant à la réponse inflammatoire et immunitaire de l'hôte (Everett & McFadden, 1999).

Les processus apoptotiques induits par les virus sont à l'origine de dérèglements cellulaires qui influencent l'évolution clinique des infections virales. L'infection par le VIH en est un exemple très représentatif. L'infection par le

VIH-1 s'accompagne d'une induction anormale de l'apoptose dans les lymphocytes T adultes, les thymocytes et les précurseurs hématopoïétiques. De plus, chez un grand nombre de patients, l'excès d'apoptose concerne toutes les populations lymphocytaires (T CD4, T CD8 et B), et le degré d'apoptose est corrélé à l'évolution de la maladie (Gougeon *et al.,* 1996). Par ailleurs, chez certains patients infectés par le VIH, l'apoptose lymphocytaire est anormalement élevée dans les ganglions lymphatiques (Amendola *et al.,* 1996), qui constituent les principaux sites de réplication du virus. Le plus surprenant est que chez les patients infectés par VIH, la majorité des cellules T subissant l'apoptose n'est pas infectée par le virus (effet « bystander ») (Finkel *et al.,* 1995). Ces observations suggèrent que la destruction des lymphocytes par le VIH est le résultat de l'activation de mécanismes cytopathogènes à la fois directs et indirects par le virus.

La régulation de l'apoptose par le VIH est d'autant plus complexe que ce virus est capable de manipuler la machinerie apoptotique à son avantage en agissant à la fois comme activateur et comme répresseur de l'apoptose. Le VIH a en effet également développé des mécanismes d'inhibition de l'apoptose pour échapper au système immunitaire de l'hôte.

Différentes études ont démontré que d'autres lentivirus, en particulier le virus de l'immunodéficience féline (FIV) et certaines souches de virus de l'immunodéficience simienne (SIV), sont également capables d'induire l'apoptose et de provoquer un syndrome de déficit immunitaire chez leur hôte naturel. Ainsi, dans le cadre d'une étude de différents modèles d'infections lentivirales chroniques de primates, les inventeurs de la présente invention ont précédemment démontré que chez le macaque rhésus infecté par la souche pathogène SIVmac251, les lymphocytes T CD4+ sont anormalement sensibles à l'apoptose (Hurtrel et al, 2005).

En revanche, dans des modèles d'infections lentivirales chroniques de primates dans lesquels l'infection, quel que soit l'isolat lentiviral en cause, n'entraîne pas de SIDA (chimpanzés expérimentalement infectés par le VIH, singes verts d'Afrique naturellement infectés par le SIVagm), il n'y a pas de programmation anormale de l'apoptose des lymphocytes T CD4+ *in vitro.* Cependant, cette absence de maladie n'est pas liée à l'absence de potentiel pathogène du virus, puisque ces virus sont capables d'induire un SIDA chez des macaques (Hurtrel et al, 2005). Ainsi, ces modèles d'infections lentivirales soulignent l'importance de facteurs propres à l'hôte qui vont déterminer soit la survenue de l'apoptose associée au développement d'un SIDA, soit l'absence de pathologie.

Un des composants majeurs de la machinerie de l'apoptose est une famille de protéases à cystéines (ou cystéines protéases) appelées caspases (de l'anglais cysteinyl aspartate-specific proteases ou cystein aspartate proteases). Les caspases ont été trouvées chez de nombreux organismes, allant de C. elegans à l'homme. Il existe à ce jour plus d'une douzaine de caspases identifiées. Ces enzymes intracellulaires ont un rôle clé dans l'apoptose, l'inflammation, l'activation et la différenciation cellulaire.

Comme d'autres protéases, les caspases sont exprimées sous la forme de pro-enzymes qui subissent une maturation protéolytique. Ces précurseurs sont exprimés constitutivement dans le cytoplasme des cellules. Les procaspases (30 à 50 kD) contiennent trois domaines : un pro-domaine N-terminal, une grande sous-unité (environ 20 kD) et une petite sous unité (environ 10 kD). L'activation implique un clivage protéolytique entre les domaines, suivi de l'association de la grande et de la petite sous unité, qui contribuent chacune aux acides aminés du site actif, pour former un hétérodimère. Les enzymes matures actives fonctionnent sous la forme d'un tétramère constitué de deux hétérodimères. Le domaine N- terminal des caspases, dont la longueur (23 à 216 acides aminés) et la séquence varient fortement, est impliqué dans la régulation de ces enzymes.

La fonction des caspases est déterminée par leur spécificité de substrat, la longueur de leur prodomaine et la séquence de ce prodomaine. Les caspases peuvent être divisées en trois groupes: les caspases inflammatoires (groupe I), les caspases initiatrices (ou régulatrices ; groupe II) et les caspases effectrices (ou exécutrices ; groupe II) (Lavrik et al., 2005). Le long prodomaine (plus de 100 acides aminés) des caspases initiatrices et des caspases inflammatoires fonctionne comme un intégrateur de signaux apoptotiques ou pro-inflammatoires. Les caspases inflammatoires, qui incluent les capsases -1, -4, -5, -11, -12, -13 et -14. Elles sont impliquées dans les processus inflammatoire et jouent un rôle central dans l'activation de certaines cytokines. Les caspases initiatrices incluent les caspases -2, -8, -9, et -10. Elle se situent en amont des cascades de signalisation apoptotiques et sont activées par des mécanismes auto-protéolytiques en réponse à des signaux pro-apoptotiques. Elles clivent et activent alors les caspases effectrices, qui se situent en aval des cascades de signalisation, ce qui permet l'amplification du signal apoptotique. Les caspases effectrices incluent les caspases -3, -6 et -7. Elles sont directement impliquées dans l'exécution ou la survenue de l'apoptose ; une fois activées par les caspases initiatrices, elles clivent de nombreuses protéines cellulaires, conduisant ainsi au démantèlement de la cellule ou à l'inactivation d'autres protéines (Thornberry and Labzebnik, 1998). Parmi les protéines inactivées par l'action de ces caspases (environ 2000 à 3000 substrats), on trouve des protéines qui protègent les cellules de l'apoptose (protéines anti-apoptotiques), telles que des protéines de la famille Bcl-2.

Les caspases, dont le domaine catalytique comprend un résidu cystéine (C), clivent leur(s) substrat(s) protéique(s) au niveau de sites consensus spécifiques contenant un résidu aspartique localisés dans la partie carboxyterminale du substrat. Elles présentent des motifs de reconnaissance du substrat et des motifs catalytiques hautement conservés (Cryns et al 1998).

Les préférences ou les spécificités de substrat de caspases individuelles ont été exploitées pour le développement de peptides qui entrent efficacement en compétition avec la liaison des caspases à leur substrat. De tels inhibiteurs synthétiques sont désormais disponibles commercialement. Certains inhibiteurs de caspase à large spectre comprennent un acide aminé unique ou une séquence d'acides aminés généralement di- à tetra- peptidique, qui est éventuellement O-méthylé(e) et qui est conjugué(e) à un groupement carboxy-terminal tel que la fluorométhyl cétone (fmk), la chlorométhyl cétone (cmk), un groupement aldéhyde (CHO) ou encore un groupement difluorophénoxy (OPh). De tels inhibiteurs ont notamment été décrits dans la demande de brevet WO 02/183341. Ces inhibiteurs de caspase sont capables de pénétrer à l'intérieur des cellules pour aller se lier irréversiblement (à l'exception des inhibiteurs avec un groupement aldéhyde dont la liaison est réversible) au site actif des caspases. Ils fonctionnent ainsi comme des leurres protéolytiques, en bloquant le clivage protéolytique des caspases, clivage qui est requis pour l'activation desdites caspases et la production d'une caspase tronquée active. Les inhibiteurs à groupement carboxy-terminal fmk ou OPh ont été formulés pour des applications in *vivo* et *in vitro.*

Deux des inhibiteurs peptidiques de caspase les plus utilisés sont les inhibiteurs Boc-D-fmk (tertioButylOCarbonyle-Asp(O-méthyl)-fluorométhylcétone; Enzyme Systems Products, CalBiochem ou R&D Systems) et z-VAD-fmk (N-benzyloxycarbonyl-Val-Ala-Asp-fluorométhylcétone; Enzyme Systems Products, CalBiochem ou R&D Systems). Les groupements Boc (tertioButylOCarbonyle) et z (N-BenzyloxyCarbonyle) servent à bloquer les séquences d'acides aminés D (Asp) ou VAD (Val-Ala-Asp), alors que le groupement fluorométhyl cétone en position carboxy-terminale facilite la perméabilité membranaire. Il a été démontré que l'inhibition de l'activation des caspases par de tels inhibiteurs prévient l'apparition des modifications morphologiques caractéristiques de l'apoptose (Chinnaiyan et al., 1997).

Un inhibiteur de caspase développé plus récemment, le Q-VD-OPh (N-(2(quinolyl)valyl-aspartyl-(2,6-difluorophénoxy)méthyl cétone; Enzyme Systems Products, CalBiochem ou R&D Systems) présente une efficacité, une stabilité et une perméabilité accrues par rapport aux inhibiteurs à groupement carboxy-terminal de type fluorométhylcétone (fmk), et une toxicité réduite, même lorsqu'il est utilisé à concentration élevée ; cet inhibiteur s'est avéré non toxique, à des doses allant jusqu'à 1g/kg de poids vivant, administrées à des souris par voie intra-péritonéale (Vera et al., 2005).

L'inhibiteur Q-VD-OPh s'est révélé fonctionnel *in vitro,* dans différents types cellulaires et *in vivo,* dans des modèles animaux, en particulier chez la souris et le rat. Par ailleurs, il a été démontré qu'il inhibe différentes caspases, en particulier les caspases -1, -3, -8, -9, -10 et -12 avec des valeurs d'IC50 allant de 25 à 400 nM. De plus, Q-VD-OPh, de même que les inhibiteurs ZVAD-fmk et Boc-D-fmk, inhibe l'apoptose de façon dose dépendante (Caserta et al., 2003).

Il a été proposé que l'inhibition de caspases ou la surexpression de la protéine anti-apoptotique Bcl-2 pourrait prévenir contre des infections virales en inhibant l'apoptose, et également perturber la production virale (Levine, 1996 ; Olsen, 1996 ; Liang, 1998 ; Wurzer 2003). Cependant, des études réalisées *in vitro* en utilisant l'inhibiteur de caspase z-VAD-fmk n'ont pas permis d'inhiber la réplication virale (Gandhi et al., 1998 ; Petit et al., 2002). Au contraire, l'administration de l'inhibiteur de caspase z-VAD-fmk à des cellules T leucémiques (CEM) ou à des cellules mononucléees du sang périphérique (PBMC) exposées au virus VIH-1 a eu pour effet d'augmenter la réplication virale (Chinnaiyan et al., 1997). Des résultats similaires ont été observés dans le cas de cellules CEM exprimant la protéine CrmA (Cytokine response modifier A) du virus de la vaccine, un inhibiteur viral de caspases inhibant notamment l'activation des caspases -1, - 6 et -8 (Chinnaiyan et al., 1997). Ceci suggère que les résultats observés dans le cas de l'inhibiteur z-VAD-fmk ne sont pas spécifiques à cet inhibiteur mais plutôt le résultat de l'inhibition de protéases pro-apoptotiques. En outre, l'inhibiteur z-VAD-fmk est capable de stimuler la réplication virale endogène dans des PBMC activées dérivées de patients VIH-1 positifs mais asymptomatiques (Chinnaiyan et al., 1997). L'ensemble de ces résultats suggère que l'apoptose pourrait servir à l'hôte à limiter la propagation du virus et que, par conséquent, des stratégies visant à inhiber la mort cellulaire pourraient avoir des conséquences délétères pour l'hôte infecté et pourraient, en particulier, contribuer à augmenter la charge virale d'un individu VIH positif. Ceci serait en accord avec le fait que de nombreux virus produisent des protéines inhibant la mort cellulaire chez l'hôte (par exemple la protéine CrmA produite par le virus de la vaccine).

Au vu de ces résultats antérieurs, la présente invention apparaît surprenante. En effet, les résultats présentés dans la présente demande démontrent que le composé Q-VD-OPh permet non seulement d'inhiber le phénotype apoptotique (inhibition des caspases, condensation et fragmentation de l'ADN) des cellules infectées par le VIH mais également d'inhiber leur mort et surtout d'inhiber la réplication virale.

L'invention a donc pour objet le composé Q-VD-OPh, pour une utilisation pour la prophylaxie et/ou le traitement d'une infection virale provoquée par un virus de l'immunodéficience humaine (VIH), en particulier chez un animal ou un humain, et plus particulièrement pour inhiber la réplication virale chez un animal ou un humain infecté par ce virus.

L'invention a également pour objet l'utilisation du composé Q-VD-OPh pour la préparation d'une composition pharmaceutique destinée à la prophylaxie et/ou au traitement d'une infection virale provoquée par un virus de l'immunodéficience humaine (VIH), en particulier chez un animal ou un humain, et plus particulièrement destinée à prévenir et/ou inhiber la réplication virale chez un animal ou un humain infecté par ce virus.

Sauf indication contraire, chaque mode de réalisation indiqué dans cette demande s'applique indépendamment et/ou en combinaison avec les autres modes de réalisation décrits.

Le composé pour son utilisation selon l'invention est le composé Q-VD-OPh non O-méthylé (N-(2(quinolyl)valyl-aspartyl-(2,6-difluorophénoxy)méthyl cétone, ayant pour formule brute C26H25F2N3O6 de formule suivante :

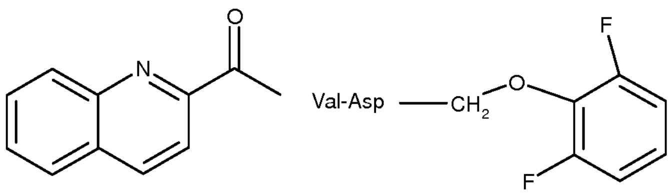

Par « agent antiviral » et « agent antirétroviral », on entend, dans la présente demande, respectivement tout agent (c'est-à-dire tout principe actif) ayant un effet antiviral ou antirétroviral. De tels agents incluent en particulier les médicaments antiviraux et en particulier antirétroviraux qui agissent sur au moins une étape de la réplication du virus. En particulier, lesdits agents peuvent permettre de prévenir, de diminuer ou d'inhiber la réplication virale.

La présente invention a également pour objet une composition, en particulier une composition pharmaceutique, comprenant au moins le composé Q-VD-OPh pour son utilisation pour la prophylaxie et/ou le traitement d'une infection virale provoquée par un virus de l'immunodéficience humaine (VIH).

Ainsi, le composé Q-VD-OPh, qui est utilisé pour la prophylaxie et/ou le traitement d'une infection virale, peut être caractérisé en ce qu'il entre dans la fabrication d'une composition, en particulier d'une composition pharmaceutique utile pour la prophylaxie et/ou le traitement d'une infection virale provoquée par un virus de l'immunodéficience humaine (VIH). Le cas échéant ladite composition comprend en outre un ou plusieurs véhicule(s), diluant(s) ou adjuvant(s) ou une de leurs combinaisons.

Le composé Q-VD-OPh, de même qu'une composition (en particulier une composition pharmaceutique) ou une combinaison (ou kit) comprenant ledit composé Q-VD-OPh, peuvent être administrés à tout animal ou à tout humain susceptible de bénéficier d'une telle administration, en particulier à tout animal ou à tout humain infecté ou susceptible d'être infecté par un virus tel que décrit dans la présente demande.

Tel qu'utilisé dans la présente demande, le terme « animal » défini tout animal non humain, en particulier tout mammifère non humain, et plus particulièrement un singe ou un chat.

Les expressions « infection virale » et « infecté par un virus » telles qu'utilisées dans la présente demande signifient que ledit animal ou ledit humain a été exposé à un virus pathogène à ARN ou à ADN et que ledit virus s'est attaché à une ou plusieurs cellules de l'hôte puis a pénétré (ou est susceptible de pénétrer) dans ladite ou lesdites cellules et a eu (ou aura éventuellement) des effets néfastes pour au moins une cellule dudit animal ou humain. En particulier une telle infection virale est capable d'évoluer vers des signes cliniques de pathologies induites ou accompagnant ladite infection. Ainsi, une « infection virale » au sens de la présente invention inclut aussi bien les phases les plus précoces de la contamination virale, de même que les phases les plus tardives ainsi que les phases intermédiaires de contamination virale. A titre d'exemple, dans le cas du VIH, l'infection évolue en plusieurs phases pouvant se succéder dans le temps. On distingue en particulier 4 phases : (1) la primo-infection correspond à la phase de séroconversion qui suit la contamination et s'accompagne (dans 50 à 75% des cas) ou non de symptômes; elle est suivie (2) d'une phase de latence, puis (3) d'une phase à symptômes mineurs, et enfin de (4) la phase d'immunodépression profonde ou stade de SIDA, qui est généralement symptomatique et s'accompagne généralement de nombreuses infections opportunistes.

Le terme « infection virale » englobe donc également tout signe clinique, symptôme ou maladie apparaissant chez un animal ou un humain (patient) suite à la contamination dudit animal ou dudit patient par un virus tel que décrit dans la présente demande. Ainsi, l'« infection virale » inclut à la fois la contamination par ledit virus et les pathologies diverses qui sont la conséquence de la contamination par ledit virus.

Les infections virales entrant dans le cadre de la présente invention sont les infections provoquées par un virus VIH, et plus particulièrement par le VIH-1 ou le VIH-2, ou un virus SIV qui incluent en particulier le syndrome d'immunodéficience acquise (SIDA).

Le terme « prophylaxie » désigne tout degré de retardement dans le moment d'apparition de signes cliniques ou de symptômes de l'infection virale, ainsi que tout degré d'inhibition de la sévérité des signes cliniques ou symptômes de l'infection virale, y compris, mais sans limitation à, la prévention totale de l'infection virale. Ceci nécessite que le composé Q-VD-OPh ou la composition ou la combinaison comprenant ledit composé Q-VD-OPh soit administré(e) à l'animal ou au patient susceptible d'être contaminé par un virus avant l'apparition de tout signe clinique ou symptôme de la maladie. L'administration prophylactique du composé Q-VD-OPh ou d'une composition ou d'un combinaison comprenant ledit composé peut avoir lieu avant que ledit animal ou humain ne soit exposé au virus responsable de l'infection virale, ou au moment de l'exposition. Une telle administration prophylactique sert à empêcher, et/ou réduire la sévérité de n'importe quelle infection subséquente.

La définition du terme «prophylaxie» ci-dessus s'applique également à l'expression « prévenir une infection ».

Par « traitement », on entend l'effet thérapeutique que produisent sur un animal ou un humain les substances actives (en particulier le ou les inhibiteur(s) de caspase) lorsqu'elles sont administrées audit animal ou audit humain au

moment de la contamination dudit animal ou humain par le virus ou après la contamination. Lorsque le composé Q-VD-OPh, une composition ou une combinaison comprenant ledit composé Q-VD-OPh est administré(e) à un animal ou à un humain après la contamination par le virus, il (elle) peut être administré(e) pendant la phase de primo-infection, pendant la phase asymptomatique ou encore après l'apparition de signes cliniques ou de symptômes de la maladie. Selon un mode de réalisation particulier, l'administration a lieu dans les 24 ou 48 heures après que ledit animal ou humain a été exposé audit virus, le plus rapidement possible.

Le terme « traitement » inclut tout effet curatif obtenu grâce au composé Q-VD-OPh ou une composition ou une combinaison comprenant ledit composé, ainsi que l'amélioration des signes cliniques ou des symptômes observés chez l'animal ou le patient, de même que l'amélioration de la condition de l'animal ou du patient. Ce terme inclut en particulier les effets obtenus en conséquence de l'inhibition de la réplication virale et/ou de l'inhibition de la mort cellulaire induite par le virus. Ainsi, le terme « traitement » couvre le ralentissement, la diminution, l'interruption, ainsi que l'arrêt de l'infection virale et/ou des conséquences néfastes de l'infection virale; un traitement n'exige pas nécessairement l'élimination complète de tous les signes cliniques de l'infection virale et les symptômes de la maladie, ni même l'élimination complète du virus.

La définition du terme « traitement » s'applique également à l'expression « traiter une infection ».

Le composé Q-VD-OPh ou une composition ou une combinaison comprenant ledit composé Q-VD-OPh peut donc être administré(e) à un animal ou à un humain qui présente des risques de développer une infection virale provoquée par un virus de l'immunodéficience humaine (VIH) (prophylaxie) ou après que la contamination par le virus a eu lieu, en particulier après la manifestation des premiers signes cliniques ou symptômes de la maladie, par exemple après que des protéines ou anticorps spécifiques dudit virus ont été détectés dans le sang de l'animal ou du patient (traitement). Selon un mode de réalisation particulier, on débute le traitement lorsque le patient présente des signes cliniques d'immunodépression ou lorsque le taux de lymphocytes T, en particulier de lymphocytes TCD4+ est inférieur à 350 par microlitre, en particulier inférieur à 200 par microlitre dans un échantillon de sang prélevé chez le patient ou chez l'animal.

Ainsi, selon un mode de réalisation particulier, le composé Q-VD-OPh ou une composition ou une combinaison ledit composé Q-VD-OPh est administré(e) à un animal ou à un humain avant que ledit animal ou humain ne soit exposé audit virus pendant l'exposition audit virus ou après l'exposition audit virus. Une administration après l'exposition au virus peut être réalisé à tout moment mais sera préférablement réalisée le plus rapidement possible après l'exposition, en particulier dans les 48 heures après que ledit animal ou humain a été exposé audit virus.

En outre, il est également possible d'envisager plusieurs administrations successives du composé Q-VD-OPh ou d'une composition ou d'une combinaison comprenant ledit composé, de façon à augmenter les effets bénéfiques du traitement. Afin d'augmenter les chances de guérison, ou du moins, prolonger l'espérance de vie de l'animal ou de l'humain, ou encore l'effet prophylactique, on peut en particulier procéder à une ou plusieurs administrations successives dudit composé Q-VD-OPh ou de ladite composition ou combinaison ou dudit kit avant que l'animal ou l'humain ne soit exposé au virus et/ou pendant l'exposition au virus et/ou après l'exposition au virus, en particulier dans les 48 heures après que ledit animal ou humain a été exposé audit virus.

Le virus entrant dans le champ d'application de la présente invention est le virus de l'immunodéficience humaine (VIH) tel que le VIH-1 ou le VIH-2 et de préférence le VIH-1.

Selon un autre mode de réalisation particulier ledit virus est un rétrovirus simien, en particulier un lentivirus simien, et plus particulièrement, un virus de l'immunodéficience simienne (SIV) tel que le virus SIVmac251 ou SIVmac239 ou tout autre virus cité dans Gordon et al.

Le composé Q-VD-OPh ou une composition ou une combinaison comprenant ce composé Q-VD-OPh peuvent être utilisés pour prévenir, diminuer et/ou inhiber la réplication virale chez un animal ou un humain infecté par un virus tel que défini ci-dessus.

Le terme « réplication virale » tel qu'utilisé dans la présente demande inclut l'ensemble des étapes du cycle de réplication du virus, en particulier les principales étapes de la réplication des rétrovirus décrites dans la présente demande, y compris l'entrée du virus dans la cellule, l'intégration du génome viral dans l'ADN de la cellule hôte et la maturation virale.

La « maturation virale » ou « maturation des virus » désigne, dans le cas des lentivirus et en particulier dans le cas des virus VIH, le processus de clivage, par la protéase virale, des polyprotéines Gag en 4 protéines de structure (p17, p24, p7 et p6) et l'assemblage de ces protéines en matrice (p17), capside (TCD4+ p24) et nucléocapside (p7). A l'issue de la phase de maturation, les virions, qui, avant le clivage, n'étaient pas matures, sont infectieux, c'est-à-dire prêts à infecter de nouvelles cellules.

La prévention ou l'inhibition de la réplication virale peut-être soit partielle, soit totale.

Selon un mode de réalisation particulier, le composé Q-VD-OPh ou une composition ou une combinaison le(s) comprenant a (ont) la capacité de prévenir, diminuer et/ou inhiber la réplication virale *in vitro.*

Avantageusement, l'effet testé *in vitro* est également obtenu *in vivo,* dans des conditions d'utilisation appropriées.

La capacité du composé Q-VD-OPh ou d'une composition ou d'une combinaison comprenant ledit composé

Q-VD-OPh à prévenir ou inhiber la réplication virale peut être évaluée par exemple, *in vitro,* par cytométrie en flux, après marquage intracellulaire d'un antigène viral tel que p24, comme décrit dans les exemples ci-après.

Selon un mode de réalisation particulier, le composé Q-VD-OPh ou une composition ou une combinaison comprenant ledit composé Q-VD-OPh est utilisé(e) pour prévenir, diminuer et/ou inhiber, la synthèse de protéines virales chez un animal ou un humain infecté par un virus tel que décrit dans la présente demande.

L'expression « protéines virales » fait référence à au moins une protéine du virus, en particulier à au moins une protéine de structure du virus. Les protéines virales dont la synthèse est susceptible d'être prévenue, ralentie, diminuée et/ou inhibée sous l'effet du composé Q-VD-OPh, incluent en particulier les protéines d'enveloppe, de capside, de nucléocapside... notamment pour les lentivirus, les protéines Gag, Pol et Env.

La prévention ou l'inhibition de la synthèse de protéines virales peut-être soit partielle, soit totale, soit partielle pour une partie des protéines virales et totale pour l'autre partie des protéines virales. Lorsqu'elle est partielle pour toutes les protéines virales ou pour certaines protéines virales, l'expression « prévenir ou inhiber la synthèse de protéines virales » signifie que sous l'effet du composé Q-VD-OPh, une ou plusieurs protéines virales sont synthétisées en quantité moindre dans la cellule hôte, et donc présentes en quantité moindre dans la cellule hôte ou dans le surnageant cellulaire, comparé à la synthèse de ces mêmes protéines virales en absence du composé Q-VD-OPh. Lorsque cette prévention ou cette inhibition de la synthèse de protéines virales est totale, la ou les protéine(s) virale(s) ne sont pas synthétisées de façon détectable.

La prévention ou l'inhibition de la synthèse des protéines peut être évaluée par exemple par immuno-transfert (Western Blot), en utilisant des anticorps spécifiques dirigés contre lesdites protéines virales, comme décrit à l'exemple B6 et en figure 6.

Le composé Q-VD-OPh ou une composition ou une combinaison comprenant ledit composé peuvent en outre être utilisés pour prévenir et/ou inhiber à la fois (i) la réplication virale, en particulier la synthèse de protéines virales et (ii) l'augmentation de la mort cellulaire, en particulier l'augmentation de la mort des lymphocytes T, et plus particulièrement des cellules T CD4+, induite par un virus tel que décrit dans la présente demande, chez un animal ou un humain infecté par ledit virus.

Le composé) Q-VD-OPh ou une composition ou une combinaison comprenant ce(s) composé(s) peuvent être utilisés en particulier pour le traitement ou la prophylaxie d'une infection virale associée à une augmentation de la mort cellulaire, en particulier une augmentation de la mort des cellules du système immunitaire et/ou des cellules neuronales, et/ou des cellules épithéliales, chez un animal ou un humain infecté par ledit virus. Les infections virales corrélées à une augmentation de la mort cellulaire des cellules T, et en particulier des cellules T CD4+, chez un animal ou un humain infecté par un virus, en particulier les infections par les virus VIH sont plus particulièrement visées.

Une « augmentation de la mort cellulaire » ou une « augmentation de l'apoptose » au sens de la présente demande signifie que le pourcentage de mort cellulaire est supérieur au pourcentage normalement observé dans le modèle cellulaire considéré. A titre d'exemple, dans le cas de cellules CEM normales (non infectées), on considérera qu'il y a une augmentation de la mort cellulaire lorsque le pourcentage de mort cellulaire sera supérieur à 1,7% (bruit de fond observé d'après Gandhi et al., 1998). A titre d'exemple également, dans une culture de lymphocytes CD4+ primaire sains activés, on considérera qu'il y a une augmentation de la mort cellulaire lorsque le pourcentage de mort cellulaire sera supérieur à 8% (bruit de fond normalement observé). Selon un mode de réalisation particulier, la mort cellulaire ou de l'apoptose augmente d'au moins 10%, au moins 20% ou au moins 40%, voire plus. Cette augmentation peut-être mise en évidence *in vitro* par n'importe quelle technique de laboratoire classiquement utilisée pour quantifier le pourcentage de mort cellulaire ou d'apoptose ; par exemple, dans le cas de la mort cellulaire, une simple numération cellulaire directement par comptage au microscope suffit. Dans le cas de l'apoptose, on peut par exemple utiliser la technique TUNEL (pour « terminal deoxyribonucleotidyl transferase (TDT)-mediated dUTP-digoxigenin nick end labeling », ou encore marquer les cellules à l'acridine orange ou mesurer la dépolarisation mitochondriale.

Par l'expression « une infection virale associée à une augmentation de la mort cellulaire » et en particulier par « une infection virale associée à une augmentation de l'apoptose », on entend une infection qui s'accompagne généralement, à plus ou moins long terme, d'une augmentation de la mort cellulaire et en particulier de l'apoptose. Cette augmentation peut être le résultat direct et/ou indirect de la contamination par un virus tel que décrit dans la présente demande.

La prévention ou l'inhibition de l'augmentation de la mort cellulaire ou de l'apoptose peut-être partielle ou totale. Lorsqu'elle est partielle, l'expression « prévenir et/ou inhiber l'augmentation de la mort cellulaire » ou « prévenir et/ou inhiber l'augmentation de l'apoptose » signifie que sous l'effet du composé Q-VD-OPh, en particulier sous l'effet d'un ou plusieurs inhibiteur(s) de caspase, le nombre de cellules mortes ou le nombre de cellules apoptotiques est réduit dans l'organisme hôte ou dans certains tissus ou pour certains types cellulaires particuliers de l'organisme hôte, par rapport à ce qu'on observe en absence du composé Q-VD-OPh. La mort cellulaire est réduite de préférence d'au moins 10%, plus préférablement d'au moins 30% et encore plus préférablement d'au moins 50%.

Selon un mode de réalisation particulier, le composé Q-VD-OPh peut être préparé sous la forme d'une composition pharmaceutique comprenant en outre un ou plusieurs véhicule(s), diluant(s) et/ou adjuvant(s) ou une de leurs

associations, ainsi que d'autres substances actives. Dans le cas d'une administration injectable, on peut notamment choisir une formulation dans une solution aqueuse, non aqueuse ou isotonique.

Le terme « véhicule » désigne, dans la présente demande, tout support (c'est-à-dire tout ce qui peut transporter au moins un principe actif) qui n'interfère pas avec l'efficacité de l'activité biologique du composé Q-VD-OPh. De nombreux véhicules sont connus dans l'état de la technique. Les véhicules utilisés peuvent être, par exemple, de l'eau, une solution saline, de l'albumine sérique, une solution Ringer, le polyéthylène glycol, des solvants miscibles dans l'eau, des sucres, des lieurs, des excipients, des pigments, des huiles végétales ou minérales, des polymères solubles dans l'eau, des agents tensioactifs, des agents épaississants ou gélifiants, des agents cosmétiques, des agents de solubilisation, des agents stabilisation, des agents conservateurs, des agents alcalinisants ou acidifiants ou une de leurs combinaisons. La formulation de tels véhicules sous forme de composition pharmaceutique est notamment décrite dans "Remington's Pharmaceutical Sciences", 18ième édition, Mack Publishing Company, Easton, Pa.

Le terme « diluant » signifie, dans la présente demande, un agent de dilution, et inclut les diluants solubles et les diluants insolubles. On utilise généralement un diluant insoluble quand le principe actif est soluble et un diluant soluble quand le principe actif est insoluble. Un principe actif « insoluble » peut être totalement insoluble en milieu aqueux ou avoir une solubilité limitée (c'est à dire une solubilité inférieure à 10mg/ml dans 250 ml d'eau à un pH de 1.0 à 7.5) en milieu aqueux. Des exemples de diluants insolubles incluent la cellulose microcristalline, la cellulose microcristalline silicifiée, l'hydroxyméthylcellulose, le phosphate de dicalcium, le carbonate de calcium, le sulfate de calcium, le carbonate de magnésium, le phosphate de tricalcium, etc. Des exemples de diluants solubles incluent le mannitol, le glucose, le sorbitol, le maltose, les dextrates, les dextrines, le dextrose, etc.

Les adjuvants susceptibles d'être utilisés dans le cadre de l'invention sont en particulier des acides nucléiques, des peptidoglycanes, des carbohydrates, des peptides, des cytokines, des hormones ou d'autres petites molécules. Lesdits adjuvants utilisés peuvent être, par exemple, les adjuvants de la famille des dinucléotides CpG non méthylés (CpG), les adjuvants de la famille des poly IC et les adjuvants de la famille du monophosphoryl lipide A (MPL) ou un de leurs analogues.

Selon un mode de réalisation préféré, les véhicule(s) ou diluant(s) ou leurs combinaisons utilisés dans l'invention sont des substances ou une combinaison de substances pharmaceutiquement acceptables. Une substance ou une combinaison de substances est dite « pharmaceutiquement acceptable » lorsqu'elle est appropriée pour une administration à un être vivant (par exemple un humain ou un animal) à des fins thérapeutiques ou prophylactiques. Elle est donc préférentiellement non toxique pour l'hôte auquel elle est administrée.

Les termes « administration » et « administrer » tels qu'utilisés dans la présente demande incluent toute administration, quelle que soit la voie d'administration choisie.

Les voies d'administration et les posologies varient en fonction d'une variété de paramètres, par exemple en fonction de l'état du patient, du type d'infection et de la sévérité de l'infection à traiter ou du composé Q-VD-OPh et des autres agents antiviraux utilisés.

Le composé Q-VD-OPh, la composition (en particulier la composition antivirale) et les constituants la combinaison sont notamment susceptibles d'être administrés à un animal ou à un humain sous forme sèche, solide (en particulier cachet, poudre, gélule, pilule, granule, suppositoire, capsule polymère ou comprimé, et plus précisément comprimé à libération accélérée, comprimé gastrorésistants ou comprimé à libération prolongée), sous forme gélatineuse ou sous la forme d'une solution ou d'une suspension liquide (en particulier sirop, solution injectable, infusible ou buvable, microvésicules, liposomes). Ces composés peuvent également se présenter sous la forme de doses sous forme sèche (poudre, lyophilisat, etc.) à reconstituer au moment de l'utilisation en utilisant un diluant approprié.

Suivant leur forme galénique, la composition (en particulier la composition antivirale), ainsi que les constituants de la combinaison peuvent être administrés par voie entérale, parentérale (intraveineuse, intramusculaire ou sous-cutanée), transcutanée (ou transdermique ou percutanée), cutané, orale, mucosale, en particulier transmuqueuse-buccale, nasale, ophtalmique, otologique (dans l'oreille), oesophagique, vaginale, rectale, ou encore les voies intragastrique, intracardiaque, intrapéritonéale, intrapulmonaire ou intratrachéale.

Par ailleurs, le composé Q-VD-OPh, la composition, ou les constituants de la combinaison peuvent être conditionnés pour une administration sous la forme d'une dose unique (monodose) ou multiple (multidose). Afin d'augmenter les effets du traitement, il est en effet envisageable de procéder à une administration sous la forme de plusieurs administrations successives, répétées à une ou plusieurs occasions, après un intervalle de temps particulier. On peut, par exemple, procéder à plusieurs administrations par jour ou par semaine.

La quantité de principe actif administrée à un animal ou à un humain est une quantité thérapeutiquement efficace. Une « quantité thérapeutiquement efficace » est une quantité suffisante pour obtenir effet significatif et en particulier apporter un bénéfice significatif à un humain ou à un animal dans le cadre d'une administration pour la prophylaxie ou le traitement tel que défini dans la présente demande. Une quantité thérapeutiquement efficace est également une quantité pour laquelle les effets bénéfiques l'emportent sur un quelconque effet toxique ou néfaste du ou des principe(s) actif(s). Une telle quantité peut correspondre à une quantité suffisante pour inhiber la réplication virale de façon significative ou pour faire disparaître, réduire, ou améliorer toute infection existante provoquée par un virus La

quantité thérapeutiquement efficace varie en fonction de facteurs tels que l'état d'infection, l'âge, le sexe ou le poids de l'animal ou de l'individu humain. Les régimes posologiques peuvent être ajustés de façon à obtenir un effet thérapeutique optimum. On peut par exemple envisager d'administrer entre 15 et 50 mg/kg de poids corporel de composé Q-VD-OPh. Plus spécifiquement, dans le cas d'un humain d'environ 60 kg, une quantité thérapeutiquement efficace de composé Q-VD-OPh peut être comprise entre 100 et 300 mg/jour, administrée en 1 à 3 prises.

La présente invention a également pour objet l'utilisation du composé Q-VD-OPh, en association avec d'autres agents antiviraux, en particulier d'autres agents antirétroviraux, pour la prophylaxie et/ou le traitement d'une infection virale provoquée par un virus de l'immunodéficience humaine (VIH). A titre d'exemple d'agents antiviraux, on citera, en rapport avec l'infection due à VIH, les drogues antirétrovirales combinées dans le cadre de la thérapie antirétrovirale hautement active (ou « HAART »).

Ainsi, une composition pharmaceutique particulière utilisée selon l'invention comprend en outre au moins un autre agent antiviral.

La présente invention a donc également pour objet une nouvelle composition antivirale, comprenant, consistant essentiellement en ou consistant en :

(i) au moins un composé Q-VD-OPh,
(ii) au moins un autre agent antiviral
pour son utilisation pour la prophylaxie et/ou le traitement d'une infection virale provoquée par un virus de l'immunodéficience humaine (VIH).

Le composé Q-VD-OPh peut donc être utilisé en association avec un agent antiviral ou plusieurs agents antiviraux, en particulier au moins deux autres agents antiviraux. Ledit autre agent antiviral ou lesdits autres agents antiviraux peuvent être en particulier des agents antirétroviraux.

L'expression « consiste essentiellement en » telle qu'utilisée dans la présente demande signifie que d'autres ingrédients ou molécules mineur(e)s peuvent être présents avec les principes actifs expressément listés, sans affecter l'activité desdits principes actifs.

Les agent antiviraux et antirétroviraux susceptibles d'être utilisés dans le cadre de la présente demande incluent en particulier :

- les inhibiteurs de transcriptase en particulier les inhibiteurs de transcriptase inverse pour les rétrovirus, qui sont destinés à agir au tout début du cycle réplicatif viral, notamment les inhibiteurs de transcriptase inverse qui sont destinés à agir avant l'intégration de l'ADN viral dans l'ADN de la cellule hôte et qui préviennent ou inhibent la synthèse d'ADN proviral à partir de l'ARN viral;
- les inhibiteurs de la protéase virale (ou antiprotéases), qui agissent généralement à la fin du cycle viral, lors de la maturation des protéines virales nouvellement synthétisées ;
- les inhibiteurs de la fusion de l'enveloppe virale avec la membrane de la cellule, qui sont destinés à bloquer la pénétration du virus dans la cellule ;
- des inhibiteurs de récepteurs ou co-récepteurs, tels que CD4 ou BOB ;
- des oligonucléotides anti-sens ;
- des inhibiteurs de l'intégrase ; et
- des molécules ciblant d'autres étapes de la multiplication virale (addressage, port d'intégration).

Selon un mode de réalisation particulier, ledit autre agent antiviral ou lesdits autres agents antiviraux consiste(nt) en au moins un inhibiteur de transcriptase et/ou au moins un inhibiteur de protéase virale.

Selon un mode de réalisation particulier, la composition antivirale pour son utilisation selon l'invention comprend, consiste essentiellement en ou consiste en :

(i) au moins un composé Q-VD-OPh,
(ii) au moins un inhibiteur de transcriptase et
(iii) au moins un inhibiteur de protéase virale.

Le terme « inhibiteur de transcriptase » tel qu'utilisé la présente demande inclut en particulier les analogues nucléosidiques, les analogues non nucléosidiques et les analogues nucléotidiques de la transcriptase inverse.

Selon un mode de réalisation particulier, l'inhibiteur de transcriptase est un inhibiteur de transcriptase inverse, en particulier un inhibiteur de la transcriptase inverse d'un virus VIH, et plus particulièrement un inhibiteur de transcriptase inverse choisi parmi le groupe constitué par :

- les inhibiteurs nucléosidiques de la transcriptase inverse du VIH, en particulier la zidovudine ou azidothymidine

(AZT), la didanosine ou ddl, la zalcitabine ou ddC, la stavudine ou d4T, la lamivudine ou 3TC, l'abacavir ou ABC, et l'emtricitabine ou FTC ;

- les inhibiteurs non nucléosidiques de la transcriptase inverse du VIH, en particulier la nevirapine, l'efavirenz et la délavirdine ; et
- les analogues nucléotidiques de la transcriptase inverse du VIH, en particulier le ténofovir ou bis-POC-PMPA.

Selon un mode de réalisation particulier, un des inhibiteurs de transcriptase utilisés est l'AZT.

Le terme « inhibiteur de protéase » tel qu'utilisé la présente demande inclut en particulier les molécules peptidomimétiques, et les molécules de type non peptidique. Des « molécules peptidomimétiques », sont des peptides qui miment le substrat naturel de l'enzyme et se fixent aux sites de liaison du substrat de la protéase, empêchant le clivage des précurseurs protéiques (par exemple Gag et Gag-Pol pour VIH ou SIV), ce qui aboutit à la production de particules virales défectueuses et non infectieuses.

Selon un mode de réalisation particulier, l'inhibiteur de protéase virale est un inhibiteur de la protéase d'un virus VIH et en particulier un inhibiteur de protéase virale choisi parmi le groupe constitué des molécules peptidomimétiques suivantes : l'Indinavir ou IDV, la Nelfinavir ou NLFN, la Saquinavir ou SQN, la Ritonavir ou RTN, l'Amprenavir, la Lopinavir. Selon un mode de réalisation particulier, un des inhibiteurs de la protéase virale du VIH utilisé est l'Indinavir.

Selon un mode de réalisation particulier, au moins un des autres agents antiviraux est un inhibiteur de fusion, en particulier un inhibiteur de la fusion du virus VIH, par exemple l'enfuvirtide. Par « inhibiteur de fusion », on entend un inhibiteur qui agit au premier stade de la réplication du virus, en empêchant la fusion entre l'enveloppe du virus et la membrane de la cellule, par exemple par inhibition compétitive.

Selon un mode de réalisation particulier, ladite composition antivirale peuvent comprendre en outre un ou plusieurs véhicule(s), diluant(s) et/ou adjuvant(s) ou une de leurs associations tels que définis dans la présente demande.

Selon un mode de réalisation particulier, au moins deux composés de la composition antivirale ou de la combinaison pour son utilisation selon l'invention agissent en « synergie ». Cela signifie que ladite composition antivirale ou ladite combinaison peut permettre d'obtenir un effet prophylactique ou thérapeutique supérieur à la somme des effets individuels de chacun des composés agissant en synergie.

Selon un mode de réalisation particulier, le composé Q-VD-OPh et au moins un des autres agents antiviraux agissent en synergie et, en particulier, le composé Q-VD-OPh agit en synergie avec au moins un des autres agents antiviraux. Les résultats présentés dans la présente invention démontrent en effet qu'un composé tel que le Q-VD-OPh est capable d'agir en synergie avec d'autres agents antiviraux, et en particulier avec l'AZT et avec l'Indinavir, pour inhiber la réplication virale et pour inhiber la mort des lymphocytes T CD4+ induite par l'infection virale.

L'utilisation du composé Q-VD-OPh, ou d'une composition ou d'une combinaison comprenant un tel composé peut donc permettre d'améliorer, de potentialiser un traitement à base d'un ou plusieurs autres agents antiviraux, en particulier un traitement antiviral peu efficace contre les virus VIH ou SIV. Le terme «potentialiser » signifie que l'utilisation du composé Q-VD-OPh permet d'obtenir un effet prophylactique ou thérapeutique supérieur à l'effet prophylactique ou thérapeutique obtenu en utilisant le ou lesdits autres agents antiviraux seuls ou en combinaison avec d'autres régimes médicamenteux.

Dans la mesure où le composé Q-VD-OPh peuvent potentialiser l'effet d'autres agents antiviraux, voire agir en synergie avec le ou lesdits autres agents antiviraux, l'utilisation du composé Q-VD-OPh peut également permettre de diminuer les doses d'autres agents antiviraux utilisées, sans diminuer l'efficacité du traitement, voire en augmentant l'efficacité du traitement.

Un autre aspect de la présente invention concerne donc l'utilisation du composé Q-VD-OPh, pour la fabrication d'un médicament destiné à potentialiser, à accroître un effet prophylactique ou thérapeutique d'un ou plusieurs autres agents antiviraux tels que définis dans la présente demande et/ou à diminuer la quantité des autres agents antiviraux administrée à un humain ou un animal.

La présente invention concerne également le composé Q-VD-OPh, pour une utilisation pour potentialiser, pour accroître un effet prophylactique ou thérapeutique d'un ou plusieurs autres agents antiviraux tels que définis dans la présente demande et/ou pour diminuer la quantité des autres agents antiviraux administrée à un humain ou un animal.

Selon un autre aspect de la présente invention, les principes actifs sont associés dans une combinaison (ou kit) pour une utilisation dans une thérapie antivirale.

Ainsi, la présente invention a également pour objet une combinaison (ou kit) comprenant, consistant essentiellement en ou consistant en :

(i) au moins un composé Q-VD-OPh et
(ii) au moins un autre agent antiviral, en particulier au moins un autre agent antirétroviral,

dans laquelle les composés (i) et (ii) sont séparés l'un de l'autre pour son utilisation pour la prophylaxie et/ou le traitement d'une infection virale provoquée par un virus de l'immunodéficience humaine (VIH). Les composés (i) et (ii) de ladite

combinaison sont tels que définis dans la présente demande. Ils peuvent être administrés au corps humain ou animal simultanément et/ou séquentiellement, séparément dans le temps. Il est possible en effet que certains constituants de ladite combinaison n'exercent pas nécessairement simultanément ou immédiatement leur activité commune.

L'expression « combinaison » (ou kit) fait référence à l'association, dans la posologie d'un traitement destiné à un humain ou un animal infecté par un virus, d'au moins deux composés appropriés pour une utilisation dans un traitement antiviral chez un animal ou un humain :

(i) au moins un composé Q-VD-OPh et
(ii) au moins un autre agent antiviral tel que défini dans la présente demande.

Les constituants (i) et (ii) forment une unité fonctionnelle du fait d'une indication commune, qui est la mise en oeuvre d'un traitement antiviral.

La combinaison peut également comprendre le composé Q-VD-OPh et plusieurs autres agents antiviraux, en particulier 2, 3, 4 ou plus.

Une telle thérapie combinée est tout particulièrement destinée à la prophylaxie et/ou au traitement d'infections virales chez un humain ou chez un animal infecté par un virus tel que défini dans la présente demande.

Le terme « simultanément » et l'expression « administration simultanée » signifient que les composés (i) et (ii) de ladite combinaison sont administrés en même temps, au même moment, à un humain ou à un animal.

Selon un mode de réalisation particulier, les composés (i) et (ii) de ladite combinaison sont présents dans deux compositions distinctes. Par « présents dans deux compositions distinctes », on entend que ledit ou lesdits et ladite ou desdites sont physiquement séparé(s). Ils sont alors mis en oeuvre, administrés séparément, sans mélange préalable, dans plusieurs (au moins deux) formes posologiques (par exemple deux capsules distinctes). Ladite combinaison correspond donc à une présentation du ou des composés (i) d'une part et du ou des composés (ii) d'autre part, dans des compositions distinctes. Le ou les composés (i) n'étant pas mélangé(s) avec le ou les composés (ii), les différents composés (i) et (ii) ne sont pas modifiés chimiquement, et peuvent être, par conséquent, administrés tel que décrits dans la ou les autorisation(s) de mise sur le marché (AMM) couvrant ce ou ces composés.

Dans le cas ou les composés (i) et les composés (ii) sont administrés séquentiellement dans le temps, l'ordre d'administration n'a pas d'importance, l'administration du ou des composés (i) pouvant précéder ou suivre l'administration du ou des composés (ii). Selon un mode de réalisation particulier, le composé (i) ou au moins un des composés (i) est administré avant d'administrer le composé (ii) ou au moins un des composés (ii). Alternativement, le composé (ii) ou au moins un des composés (ii) peut être administré avant d'administrer le composé (i) ou au moins un des composés (i).

L'expression « administration séquentielle » signifie que ledit ou un desdits composés (i) et ledit ou un desdits composés (ii) de la combinaison ou du kit pour son utilisation selon l'invention sont administrés non pas simultanément mais séparément dans le temps, l'un après l'autre.

Le terme « précéder » ou « précédant » est utilisé lorsqu'un composé (ou plusieurs composés) de la combinaison ou du kit pour son utilisation selon l'invention est administré quelque minutes ou plusieurs heures, voire plusieurs jours avant l'administration du ou des autre(s) composé(s) de ladite combinaison ou dudit kit. A l'inverse, le terme « suivre » ou « suivant » est utilisé lorsqu'un composé (ou plusieurs composés) de la combinaison ou du kit pour son utilisation selon l'invention est administré quelque minutes ou plusieurs heures, voire plusieurs jours après l'administration du ou des autre(s) composé(s) de ladite combinaison ou dudit kit.

En outre, selon un mode de réalisation particulier, les composés (i) et (ii) de la combinaison ou du kit pour son utilisation selon l'invention sont formulés pour une administration à une ou plusieurs heures d'intervalle, de préférence à 1, 2, 3 ou 4 heure(s) d'intervalle, plus préférablement à 1 ou 2 heures d'intervalle, plus préférablement encore à 1 heure d'intervalle.

Le ou les composés (i) et le ou les composés (ii) de la combinaison peuvent être formulés pour faciliter leur prise et en particulier, peuvent être formulés avec un ou plusieurs véhicule(s), diluant(s) ou adjuvant(s) tels que définis ci-dessus ou une de leurs associations.

Par ailleurs, le ou les composés (i) et le ou les composés (ii) de la combinaison ou du kit pour son utilisation selon l'invention peuvent être administrés par les mêmes voies d'administration ou, au contraire, par des voies d'administrations distinctes. Les formes galéniques et les voies d'administration envisageables sont celles décrites ci-dessus.

Selon un mode de réalisation particulier, ledit autre agent antiviral ou lesdits autres agents antiviraux consiste(nt) en au moins un inhibiteur de transcriptase, notamment de transcriptase inverse tel que défini dans la présente demande et/ou au moins un inhibiteur de protéase virale tel que défini dans la présente demande.

De préférence, on utilise au moins deux autres agents antiviraux. Ainsi, selon un mode de réalisation particulier, la composition pour son utilisation selon l'invention comprend :

(i) au moins le composé Q-VD-OPh,
(ii) au moins un inhibiteur de transcriptase, et

(iii) au moins un inhibiteur de protéase virale.

La présente invention a également pour objet une composition antivirale ou une combinaison selon l'invention pour une utilisation comme médicament, en particulier comme agent antiviral et plus particulièrement comme agent antirétroviral. Plus précisément, ladite composition antivirale ou ladite combinaison peut être utilisée pour la prophylaxie et/ou le traitement d'une infection virale, en particulier une infection provoquée par un virus tel que défini dans la présente demande et plus particulièrement pour inhiber la réplication virale, chez un mammifère ou un humain.

La présente invention a également pour objet l'utilisation d'une composition antivirale ou d'une combinaison selon l'invention pour la fabrication d'une composition pharmaceutique destinée à la prophylaxie et/ou le traitement d'une infection virale, provoquée par un virus tel que défini dans la présente demande, chez un mammifère ou un humain.

Est décrite ci-après une méthode de traitement d'un animal ou d'un humain infecté par un virus tel que décrit dans la présente demande, ladite méthode comprenant au moins une étape d'administration du composé Q-VD-OPh, de la composition antivirale pour son utilisation selon l'invention ou des constituants de la combinaison pour son utilisation selon l'invention.

Ladite méthode de traitement est, en particulier, appropriée pour et destinée à une utilisation pour la prophylaxie et/ou le traitement d'une infection virale, en particulier chez un humain ou un animal infecté par un virus tel que défini dans la présente demande.

Plus précisément, ladite méthode de traitement peut être utilisée pour prévenir et/ou inhiber la réplication virale chez un animal ou un humain infecté par un virus.

En outre, ladite méthode de traitement peut être utilisée pour prévenir et/ou inhiber l'augmentation de la mort cellulaire chez un animal ou un humain infecté par un virus, en particulier l'augmentation de la mort des lymphocytes T, et plus particulièrement l'augmentation de la mort des lymphocytes T CD4.

On considère que la méthode de traitement atteint l'effet thérapeutique escompté si elle permet d'obtenir une diminution d'au moins 10%, de préférence 30%, et plus préférablement d'au moins 50% de la réplication virale chez l'animal ou l'humain traité. L'effet thérapeutique peut également être défini par rapport aux standards cliniques développés pour mesurer les effets bénéfiques des médicaments sur l'infection par le VIH. Une drogue a un effet bénéfique si elle divise par deux la charge virale plasmatique chez l'homme. Une drogue a un effet super bénéfique si elle divise par 100 la charge virale plasmatique chez l'homme.

## DESCRIPTION DES DESSINS

**Figure 1** : **Immuno-empreinte (Western Blot) mettant en évidence l'efficacité de Q-VD-OPh à inhiber l'activation des caspases -3 et -8.** Des lymphocytes T $CD4^+$ ont été infectés par le virus VIH-1 (incubation : 2 heures) ou non, puis stimulés par la Concanavaline A et l'IL-2 (incubation : 2 heures). Après la stimulation, l'inhibiteur Q-VD-OPh a été ajouté à la concentration finale de 10 $\mu$M à la culture cellulaire puis ajouté également 36 heures après l'infection (J3) et 96 heures après infection (J4). L'immuno-empreinte a été réalisée au 6$^{ème}$ jour post-infection. Les caspases -3 et -8 ont été détectées en utilisant les anticorps anti-caspase indiqués ci-dessus. **NI :** cellules contrôle (cellules T $CD4^+$ non infectées par le VIH-1 et non traitées au Q-VD-OPh) ; **VIH** : cellules T $CD4^+$ infectées par le VIH-1 mais non traitées au Q-VD-OPh ; **VIH+QVD** : cellules T $CD4^+$ infectées par le VIH-1 puis cultivées en présence de 10 $\mu$M de Q-VD-OPh. L'actine sert de contrôle de chargement du gel en protéines. Le marqueur de poids moléculaire RPN 800 (Amersham) a été utilisé.

**Figure 2** : **Analyse des lymphocytes T CD4+ par cytométrie en flux au 5ème jour après infection par le VIH-1 et après stimulation par la Concanavaline A et l'IL-2. A.** Analyse de la taille et de la granulométrie des lymphocytes T CD4+. **B.** Détermination du pourcentage de cellules CD4+ présentant une atteinte mitochondriale **($\Delta\Psi$m). C.** Détermination du pourcentage de cellules CD4+ infectées par le VIH après un marquage intracellulaire de l'antigène virale p24. **NI** : lymphocytes T CD4+ non infectés; **VIH :** lymphocytes T CD4+ infectés par le VIH-1; **VIH Q-VD-OPh** : lymphocytes T CD4+ infectés par le VIH-1 (incubation : 2 heures), activés par la ConA et l'IL2 (incubation 2 heures), puis incubés avec du Q-VD-OPh (10 $\mu$M finale) ; le Q-VD-OPh est ajouté à 36 heures (jour 3) et 96 heures (jour 4) après le début de l'infection.

**Figure 3 : Immuno-empreinte (Western Blot) mettant en évidence l'effet de Q-VD-OPh sur les dommages mitochondriaux résultant de la réplication virale.** Des cellules T CD4+ primaires ont été infectées avec VIH-Lai et stimulées avec un cocktail Con A/IL-2, puis traitées ou non avec 10 $\mu$M de Q-VD-OPh **(QVD)** et/ou 10 $\mu$M de pepstatine A **(PA)** tout de suite après la stimulation, puis 36 heures (jour 3) et 96 heures (jour 4) après l'infection. Au 5eme jour post-infection, les cellules ont été fractionnées en deux parties : la fraction membranaire et la fraction cytosolique. La relocalisation des facteurs apoptogéniques Cytochrome c **(Cyt c),** Smac/Diablo, et Endonucléase G (EndoG) a ensuite été évaluée par immuno-transfert (Western Blot). Les protéines Cytochrome c, Smac/Diablo,

et EndoG ont été détectées en utilisant les anticorps indiqués ci-dessus. Les protéines Cox IV et l'actine détectées respectivement grâce aux anticorps anti sous unité IV (clone 1068 Molecular Probe) et aux anticorps antiactine (SIGMA) ont été utilisées respectivement comme contrôle de la procédure de fractionnement et comme contrôle de chargement du gel en protéines.

**Figure 4** : **Représentation graphique illustrant l'effet de Q-VD-OPh sur la réplication du virus VIH-1. A.** Détermination du pourcentage de lymphocytes T CD4+ infectés par le VIH-1 par cytométrie en flux, après marquage intracellulaire de l'antigène viral p24. **B.** Dosage par ELISA de l'antigène viral p24 dans les surnageants de culture. Toutes les cellules ont été stimulées par la Con A et l'IL2 dans les 24 premières heures après l'infection. Les paramètres ont été mesurés au 6ème jour post-infection. **NI** : lymphocytes T CD4+ non infectés; **VIH** : lymphocytes T CD4+ infectés par le VIH-1; **VIH+QVD et VIH+PA+QVD** : lymphocytes T CD4+ infectés par le VIH-1, auxquels on a administré, au 3ème jour post-infection, puis tous les jours, du Q-VD-OPh (QVD) ou du Q-VD-OPh (QVD) et de la pepstatine A (PA) respectivement.

**Figure 5** : **Le moment auquel Q-VD-OPh est administré aux lymphocytes T CD4+ influence l'inhibition de la mort cellulaire et l'inhibition de la réplication virale.** Des lymphocytes T CD4+ ont été infectés ou non avec VIH-Lai et stimulés avec un cocktail Con A/IL-2, puis traités ou non avec 10 μM de Q-VD-OPh et/ou 10 μM de pepstatine A. On a déterminé, par cytométrie en flux, le pourcentage de lymphocytes T CD4+ infectés par le VIH après un marquage intracellulaire de l'antigène p24 **(A)** et le pourcentage de lymphocytes T CD4+ morts **(B)** au 5ème jour après l'infection par le VIH-1. Les paramètres ont été mesurés au 5ème jour post-infection. **NI** : lymphocytes T CD4+ non infectés par le VIH-1; **VIH** : lymphocytes T CD4+ infectés par le VIH-1 ; **Q-VD** : lymphocytes T CD4+ infectés par le VIH-1, auxquels on a administré du Q-VD-OPh et de la pepstatine A **(PA);** dans le premier cas **(Before),** le Q-VD-OPh et PA ont été administré entre 1 et 2 heures avant l'infection par le VIH-1 et la stimulation par la Concanavaline A et l'IL-2 puis à J3 et J4 postinfection, à une concentration finale de 10 μM. Dans le deuxième cas **(After),** le Q-VD-OPh et PA ont été administré tout de suite après l'infection par le VIH-1 et à J3 et J4 postinfection à différentes concentrations finales (0,5 ; 1 ; 2 ; 5 ; 10 et 20 μM).

**Figure 6 : Immuno-empreinte (Western Blot) mettant en évidence l'effet inhibiteur de Q-VD-OPh sur l'expression d'un ensemble de protéines du VIH-1 dans des cellules CD4+ primaires.** Des extraits protéiques réalisés au 6ème jour de culture à partir de lymphocytes T CD4+ non infectés **(NI)** ou infectés par le Virus VIH-1 puis cultivés en absence de Q-VD-OPh **(VIH)** ou en présence de Q-VD-OPh **(VIH + Q-VD-OPh),** ont été fractionnées en trois parties : les fractions cytosoliques **(cytosol),** les fractions membranaires solubles **(solubles)** et les fractions membranaires insolubles **(insolubles).** Le Q-VD-OPh a été ajouté à une concentration finale de 10μM, tout de suite après l'infection puis à 36 heures **(J3)** et 96 heures **(J4)** post infection. Les protéines spécifiques du VIH ont été détectées en utilisant un mélange de sera de patients VIH+. Elles sont indiquées à droite de l'immuno-empreinte. Le marqueur de poids moléculaire RPN 800 (Amersham) a été utilisé.

**Figure 7 : Représentation graphique illustrant l'effet de Q-VD-OPh sur des souches primaires de VIH-1.** Dans tous les cas **(A, B ou C),** les lymphocytes T CD4+ ont été stimulés avec de la concanavaline A et de l'IL2 (la stimulation étant réalisée avant ou pendant l'administration de Q-VD-OPh) et le Q-VD-OPh a été ajouté à une concentration finale de 10μM, tout de suite après l'infection puis à 36 heures **(J3)** et 96 heures **(J4)** post infection. A. Analyse par cytométrie en flux de la réplication virale chez des lymphocytes T CD4+ infectés avec le virus VIH-1 Lai ou avec le sérum d'un patient VIH+ chronique (souche à double tropisme X4/R5), puis cultivés en absence ou en présence **(+ Q-VD)** de Q-VD-OPh. Le pourcentage de cellules CD4+ infectées par le VIH a été déterminé après un marquage intracellulaire de l'antigène viral p24 au 5ème jour post-infection dans le cas du virus VIHLai et au 6ème jour post-infection dans le cas du sérum. **PE** : phycoérythrine, fluorochrome (émission à 578 nm) couplé à l'anticorps anti-p24. **B.** Quantification de la réplication virale (à gauche) et de la mort cellulaire, par mesure de la dépolarisation mitochondriale (à droite) chez des lymphocytes T CD4+ infectés avec le sérum du patient VIH+ chronique. **C.** Analyse de la réplication de cinq isolats viraux provenant de patients VIH+, de tropisme R5, en absence (-) ou en présence (+) de Q-VD-OPh ou d'une concentration finale de 1 μM de didanosine (ddI, SIGMA), un inhibiteur de la transcriptase inverse également appelé Vivex EC® (Bristol-Myers Squibb).

**Figure 8 : Représentation graphique illustrant l'effet inhibiteur de Q-VD-OPh sur la réplication du virus SIVmac251 et la formation de syncitia.** La lignée cellulaire CEMx174 a été infectée avec 200 AID50 (dose infectieuse nécessaire pour avoir 50% d'animaux infectés) de la souche SIVmac251. Deux heures après l'infection, la lignée cellulaire a été traitée avec différentes concentrations d'inhibiteur Q-VD-OPh (concentration finale de 20, 10 ou 2,5 μM) ou non (0 μM). Aux 4ème et 5ème jours post-infection, on a évalué la production virale dans le surnageant de culture par RT-PCR (Taqman) et le nombre de syncytia au microscope optique.

**Figure 9 : Représentation graphique mettant en évidence un effet synergique de Q-VD-OPh avec l'AZT et l'Indinavir.** Des cellules T CD4+ primaires infectées par le virus VIH-Lai puis stimulées avec de la concanavaline A et de l'IL-2 ont été traitées 96 heures après l'infection avec différentes concentrations de Q-VD-OPh (0, 0,1, 1 et 10 μM) en absence ou en présence d'azidothymidine **(AZT;** 0.1 μM), ou d'Indinavir (**IND**; 1 μM). L'inhibition de la mort cellulaire a été quantifiée par cytométrie en flux au 5ème jour post-infection. Elle est exprimée de la façon

suivante :

(% de mort cellulaire induite par le VIH en absence de traitement - % de mort cellulaire induite par le VIH en présence de traitement) / (% de mort cellulaire induite par le VIH en absence de traitement - % de mort cellulaire dans le contrôle) x 100.

**Figure 10.** Analyse par cytométrie de flux de la chute du potentiel transmembranaire mitochondriale (% $\Delta\varphi$m low) à partir de cellules Jurkat incubées en absence ou en présence de différentes concentrations d'anti-CD95 (**A**) et en absence ou en présence de 0.25 $\mu$g/ml d'anti-CD95 et de différents inhibiteurs de caspases (**B**). **C.** Analyse par immunoempreinte d'extraits de cellules Jurkat traitées en présence ou non d'anti-CD95 et de différents inhibiteurs de caspases.

**Figure 11.** Analyse par cytométrie en flux de la protéine virale p24 interne (**A**) et de la chute du potentiel transmembranaire mitochondriale (% $\Delta\varphi$m low ; **B**) aux $5^{ème}$ (J5) et 7ième (J7) jours post-infection chez des cellules T CD4+ primaires infectées par la souche VIH-Lai, après stimulation par la Concanavaline A et l'IL-2 et en présence ou non de 10 $\mu$M de différents inhibiteurs de caspases.

**Figure 12.** Analyse par immunoempreinte de la quantité de protéines virales du VIH produites à partir d'extraits de cellules primaires T CD4 infectées par la souche VIH-Lai en présence ou non de différents inhibiteurs de caspases.

**Figure 13.** Analyse par cytométrie de flux de la prolifération lymphocytaire après 4 et 5 jours de stimulation par 1$\mu$g/ml d'anti-CD-3, en présence ou en absence d'anti-protéases du VIH ou de QVD-OPH.

**Figure 14.** Analyse de la chute du potentiel transmembranaire mitochondriale (% $\Delta\varphi$m low) par cytométrie de flux à partir de cellules stimulées par 1$\mu$g/ml d'anti-CD-3 en présence ou en absence de différents anti-protéases du VIH ou de QVD-OPH.

**Figure 15.** Analyse de la chute du potentiel transmembranaire mitochondriale (% $\Delta\varphi$m low) par cytométrie de flux chez des monocytes et les lymphocytes en présence des différents anti-protéases du VIH ou de QVD-OPH.

## EXEMPLES

## EXEMPLE 1 : ANALYSE DES PROPRIETES DE Q-VD-OPH

### A. MATERIEL ET METHODES

***Anticorps***

***Pour les Immunoempreintes (Western Blot):*** anticorps polyclonaux de lapin anti-Smac/Diablo ($\Psi$ProSci), anti-Endonucléase-G ($\Psi$ProSci), anti-caspase-3 (Stressgen), anti-actine (Sigma), anticorps monoclonaux anti-caspase-8 (Cell Signaling), anti-Cytochrome c clone 7H8.2C12 (BD Pharmingen), anti-Cox IV, sous-unité IV, clône 10G8 (Molecular Probes).

***Pour la cytofluorométrie:*** anticorps monoclonal anti-p24, clône KC57-RD1 (Beckman coulter).

***Inhibiteurs synthétiques***

***Inhibiteur de la cathepsine D:*** pepstatine A (Sigma).

***Inhibiteur de caspase à large spectre:*** Q-VD-OPh sous forme non O-méthylée (N-(2(quinolyl)valyl-aspartyl-(2,6-difluorophénoxy)méthyl cétone ; Enzyme Systems Products, MP Biomedicals).

***Inhibiteur de la transcirptase inverse:*** didanosine (ou ddl) ou Videx EC® (Bristol-Myers Squibb).

***Isolement de cellules $CD4^+$ et conditions de culture***

Des cellules mononuclées de sang périphérique obtenues de patients volontaires sains (Etablissement Français du sang) ont été isolées sur couches de Ficoll (Petit et al., 2002). La majorité des cellules adhérentes ont été éliminées par incubation dans des boîtes de culture en plastique. Les cellules $CD4^+$ circulantes ont été sélectionnées négativement en utilisant un Kit d'isolement des cellules $CD4^+$ selon les instructions du fournisseur (MACS, CD4 T cell isolation kit II; Miltenyi Biotech, Paris, France). La pureté de la population $CD4^+$ isolée, déterminée par cytométrie de flux, était $\geq$ 96%. Des monocytes récupérés des boîtes d'adhérence ont été rajoutés aux cellules $CD4^+$ purifiées à un pourcentage final de 6%. La composition du milieu de culture qui a été utilisé est la suivante : RPMI 1640, 10% de sérum de veau foetal, 2 mM de glutamine, 1mM de pyruvate, 50 unités/ml de pénicilline et 50 $\mu$g/ml de streptomycine.

***Mesure de la réplication virale***

Les cellules CD4$^+$ ont été incubées pendant 2 heures à 37°C en présence de 10ng/ml de virus VIH-1 de la souche Lai ou de 50ng/ml de souches primaires du virus VIH-1. Après 2 lavages, les cellules ont été resuspendues dans un milieu complet en présence de 5 μg/ml de concanavaline A (Con A) et 10 mg/ml d'interleukine 2 (IL 2). L'antigène VIH p24, témoin de la charge virale, a été mesuré dans les surnageants de culture cellulaire par un test ELISA (Abbott). L'antigène p24 intracellulaire a été déterminé par cytométrie en flux à l'aide d'un anticorps spécifique (KC57, Coulter Corp), et après perméabilisation des cellules utilisant le réactif de pérméabilisation Intraprep (Coulter Corp.).

***Mesure de la mort cellulaire et analyse par cytométrie de flux***

Pour évaluer le changement du potentiel transmembranaire de la membrane interne de la mitochondrie (Δφm), les cellules CD4$^+$ ont été marquées pendant 15 min. à 37°C avec 40 nM de $DIOC_6$ (3-3'-Diethyloxacarbocyanine). Les cellules mortes présentent une diminution de l'intensité de marquage. La taille et la morphométrie ont été également déterminées. Les cellules apoptotiques/mortes ont été dénombrées par microscopie en lumière blanche, en se basant sur la morphologie anormale de la cellule et/ou l'absorption du bleu trypan.

***Immunoempreinte (Western Blot)***

Des extraits de 20 μg de chacune des fractions cytosoliques et mitochondriales ont été bouillis pour 5 min dans du tampon Laemmli contenant 2% SDS et 10% 2-β-mercaptoéthanol, puis migrés sur des gels de gradients de polyacrylamide de 10-20% (Bio-Rad). Après transfert des protéines sur une membrane de difluoride polyvinylidene (Bio-Rad), les immunoempreintes ont été incubées avec les anticorps primaires et après secondaires qui sont couplés à la peroxidase raifort (horseradish) obtenus d'Amersham Biosciences (Orsay, France). En suite, ils ont été développés et révélés par chemiluminescence (ECL from Amersham or West Femto from Pierce) utilisant une caméra CCD (Fuji LAS-1000plus) et le logiciel L process de Science Lab (Isochem, Paris, France).

***Fractionnement subcellulaire***

Les fractions cytosoliques et mitochondriales ont été obtenues par la technique de fractionnement subcellulaire basée sur la perméabilisation sélective par la digitonine selon Foghsgaard *et al.* {Foghsgaard, 2001}. Brièvement, $10^7$ de cellules ont été lavées deux fois dans du PBS et incubées pour 5 min sur la glace avec 100 μl de tampon d'extraction (35 μg/ml digitonine, 250 mM sucrose, 137 mM NaCl, 70 mM KCl, 4.3 mM $Na_2HPO4$, 1.4 mM $KH_2PO4$, 2 mM EDTA, pH 7.2) supplémentées d'un cocktail d'inhibiteur de protéases ("Complete" from Roche Applied Science, Penzberg, Germany). Les extraits ont été centrifugés à 300g pour 5 min, et le surnageant résultant a été recentrifugé de nouveau à 10,000 g pour 10 min à 4°C pour éliminer les débris. Le surnageant final, nommé fraction cytosolique, a été stocké à - 80°C. La pellette a été solubilisée dans 100 μl de tampon de lyse mitochondriale (50 mM Tris pH 7.4, 150 mM NaCl, 2.5 mM EDTA, 2.5 mM EGTA, 0.5% NP40, 0.2% Triton X100) supplémenté du "Complete", cocktail d'inhibiteur de protéases de Roche pendant 30 min sur la glace à 4°C, suivi d'une centrifugation à 10 000 g pour 30 min à 4°C pour obtenir la fraction mitochondriale dite « soluble ». La concentration des protéines des fractions cytosoliques et mitochondriales a été déterminée par la méthode de l'acide bicinchonique (BCA) (Bio-Rad).

**B. RESULTATS**

**B-1. Q-VD-OPh inhibe l'activation des caspases -3 et -8.**

L'efficacité de Q-VD-OPh à inhiber l'activation des caspases -3 et -8 a été analysée par immunoempreinte (Western Blot). Des lymphocytes T CD4$^+$ en culture ont été infectés par le virus VIH-1 puis 2 heures après l'infection stimulés par la Concanavaline A et l'IL-2. A la suite de la stimulation, 10 μM d'inhibiteur Q-VD-OPh a été ajouté à la culture cellulaire. Le Q-VD-OPh a été ajouté à nouveau 36 heures (3 jours), puis 96 heures (4 jours) après l'infection toujours à la concentration finale de 10 μM. L'extraction protéique et l'immuno-empreinte ont été réalisées au 6$^{ème}$ jour post-infection.

Les résultats obtenus (voir figure 1) montrent qu'en l'absence d'infection virale, les caspases -3 et -8 se trouvent essentiellement sous les formes p32 et p55 respectivement, qui correspondent aux formes inactives (pro-formes) des caspases -3 et - 8. On trouve également les formes intermédiaires p20 pour la caspase-3 et p44, p26 et p20 pour la caspase-8 (Alam et al., 1999 ; Petit et al., 2002).

Lorsque les cellules CD4+ ont été infectées par le VIH-1 puis cultivées en l'absence d'inhibiteur Q-VD-OPh, on constate, au 6ème jour post-infection, que les pro-formes p32 et p55 ne sont plus que faiblement détectées, alors

que les formes intermédiaires sont présentes en quantité plus importante. De plus, on détecte la présence des formes p17 et p18, qui correspondent aux formes actives (formes apoptogènes) des caspases -3 et -8 respectivement. Ceci reflète l'activation par protéolyse des caspases -3 et -8 suite à l'infection virale.

Lorsque les cellules CD4+ ont été infectées par le virus VIH-1 et cultivées en présence d'inhibiteur Q-VD-OPh, on observe une inhibition de la dégradation protéolytique des caspases -3 et -8; les formes actives p17 et p18 ne sont pas détectées, alors que les formes intermédiaires et les pro-formes p32 et p55 sont détectées beaucoup plus fortement que lorsqu'on n'administre pas d'inhibiteur aux cellules infectées par le VIH. Par conséquent, l'utilisation de l'inhibiteur Q-VD-OPh a pour effet d'inhiber la dégradation protéolytique des pro-formes des caspases -3 et -8 et ainsi de bloquer l'activation des caspases -3 et -8.

**B-2. Q-VD-OPh inhibe la mort cellulaire provoquée par l'infection par le VIH-1.**

Les inventeurs ont évalué l'effet du composé Q-VD-OPh sur la mort cellulaire provoquée par l'infection par le VIH-1 et la réplication virale en analysant des cellules T CD4+ infectées par le virus VIH-1 puis stimulées par la Concanavaline A et l'IL-2 et incubées ou non en présence de Q-VD-OPh pendant 5 jours (Figure 2). L'analyse de la taille et de la granulométrie des cellules T CD4+ (Figure 2A) ainsi que du pourcentage de cellules CD4+ présentant une dépolarisation mitochondriale, caractéristique d'une mort cellulaire (Figure 2B), démontre que l'infection par le VIH-1 s'accompagne d'une forte augmentation de la mort cellulaire des cellules T CD4+ (63,1% contre 13,3 pour les cellules non traitées. En revanche, lorsque les cellules T CD4+ ont été incubées avec l'inhibiteur Q-VD-OPh après avoir été infectées par le VIH-1, on n'observe qu'une légère augmentation de la mort cellulaire des cellules T CD4+ (19,8% contre 13,3% pour les cellules non traitées. Ces résultats démontrent que Q-VD-OPh est un puissant inhibiteur de la mort cellulaire résultant de l'infection par le VIH-1, contrairement à d'autres inhibiteurs de caspase à large spectre, tel que zVAD-kmk, qui prévient le phénotype apoptotique (condensation et fragmentation de la chromatine nucléaire) dans des cellules infectées par le VIH mais n'empêche pas la dépolarisation mitochondriale ni la mort cellulaire (Petit et al., 2002)

**B-3. Q-VD-OPh inhibe les dommages mitochondriaux apoptogènes provoqués par la réplication virale.**

Les inventeurs ont analysé par immuno-transfert (Western Blot) l'effet de Q-VD-OPh sur la libération de facteurs mitochondriaux apoptogènes provoquée par la réplication virale. Des cellules T CD4+ primaires ont été infectées avec le virus VIH-Lai puis stimulées avec un cocktail concanavaline A/IL-2 avant d'être traitées avec du Q-VD-OPh (10 $\mu$M) et/ou de la pepstatine A (10 $\mu$M). Au jour 5 après infection, les cellules ont été fractionnées en deux parties : (i) la fraction membranaire mitochondriale et (ii) la fraction cytosolique, qui est susceptible de contenir des facteurs mitochondriaux libérés suite à la perméabilisation de la membrane mitochondriale provoquée par l'infection virale.

L'analyse de la localisation des facteurs apoptogènes Cytochrome C, Smac/Diablo, et Endonucléase G (EndoG) (Figure 3) montre la présence de ces facteurs apoptogènes dans la fraction cytosolique des cellules infectées par le VIH-Lai, alors que dans les cellules non infectées, ils sont localisés dans la fraction membranaire mitochondriale, ce qui est l'indicateur de dommages mitochondriaux. Ceci suggère que la mort massive des cellules T CD4+ observée suite à une infection par le VIH est le résultat d'un mécanisme de mort cellulaire qui passe par une perte de la perméabilité de la membrane mitochondriale et un relargage dans le cytosol de facteurs apoptogènes.

Par ailleurs, alors que l'utilisation de la pepstatine A ne semble pas avoir d'effet au bout de 5 jours sur les dommages mitochondriaux provoqués par l'infection virale, l'utilisation de Q-VD-OPh permet de réduire très fortement la présence des facteurs apoptogènes Cytochrome C, Smac/Diablo, et EndoG dans la fraction cytosolique ; la présence de Cytochrome C est même quasi-nulle. Ces résultats démontrent que Q-VD-OPh permet de réduire considérablement les dommages mitochondriaux provoqués par la réplication virale.

**B-4. Q-VD-OPh inhibe la réplication du virus VIH-1.**

Afin de déterminer l'effet du composé Q-VD-OPh sur la réplication virale, la réplication du virus VIH-1 en présence ou en absence de Q-VD-OPh a été évaluée via la quantification du nombre de lymphocytes TCD4+ exprimant l'antigène p24 dans des lymphocytes T CD4+ infectés par le VIH et stimulés par la ConA et l'IL-2. L'analyse du pourcentage de lymphocytes T CD4+ infectés par le VIH-1 aux 5ème et 6ème jours post-infection (Figures 2C et 4A respectivement) et du dosage de l'antigène viral p24 dans les surnageants de culture au 6ème jour post-infection (Figure 4B) démontrent que le composé Q-VD-OPh diminue de plus de 75% la réplication virale. L'inhibition de la réplication virale est encore plus forte lorsque les lymphocytes T CD4+ sont traités à la fois avec le composé Q-VD-OPh et avec un autre inhibiteur de protéase, la pepstatine A (inhibiteur de la pepsine).

Les figures 2C et 4A représentent le comptage des cellules exprimant l'antigène viral p24 par marquage immunologique et cytométrie en flux. Cinq jours après l'infection, 72,6% des cellules expriment l'antigène viral et sont donc infectées. Si l'infection est suivie de l'addition de Q-VD-OPh, cinq jours après l'infection, seulement 18% des

cellules sont infectées.

**B-5. L'effet inhibiteur de Q-VD-OPh sur la mort cellulaire provoquée par l'infection virale et sur la réplication virale est d'autant plus fort que celui-ci est administré précocément.**

De façon à déterminer si le moment auquel le composé Q-VD-OPh est administré a un impact sur l'inhibition de la mort cellulaire provoquée par l'infection par le VIH-1 et sur l'inhibition de la réplication virale, Q-VD-OPh a été administré à des lymphocytes T CD4+ en culture soit avant l'infection par le VIH-1 soit après. Dans chaque cas, on a déterminé, par cytométrie en flux, au 5ème jour post-infection, le pourcentage de lymphocytes T CD4+ infectés par le virus par un marquage intracellulaire de l'antigène p24 (Figure 7A) et le pourcentage de lymphocytes T CD4+ morts (Figure 7B).

Les résultats obtenus en utilisant des concentrations croissantes de Q-VD-OPh (0,5 ; 1 ; 2 ; 5 ; 10 et 20 μM) administrées aux lymphocytes T CD4+ après l'infection par le VIH-1 démontrent que l'inhibition de la réplication virale et l'inhibition de la mort cellulaire provoquée par l'infection virale sont dose dépendantes.

Le fait qu'une forte concentration d'inhibiteur (20 μM) s'accompagne d'une forte inhibition de la mort cellulaire en réponse à l'infection virale souligne également que non seulement l'inhibiteur Q-VD-OPh n'est absolument pas toxique pour les cellules T CD4+ mais que, au contraire, il favorise la survie des cellules T CD4+ infectées par le virus VIH-1. De plus, il est possible que le Q-VD-OPh bloque la mort des cellules CD4+ non infectées (effet bystander ; Hurtrel et al., 2005).

En outre, lorsque le Q-VD-OPh est ajouté avant l'infection par le VIH-1 (à la concentration finale de 10 μM), l'inhibition de la réplication virale et l'inhibition de la mort cellulaire sont encore plus importantes que lorsque le Q-VD-OPh est ajouté après l'infection par le VIH-1 (en concentration de 0,5 à 20 μM). Ceci démontre que l'inhibiteur Q-VD-OPh a un effet d'autant plus fort sur l'infection virale et sur les conséquences de l'infection virale qu'il est administré précocement. Ainsi, l'inhibiteur Q-VD-OPh pourrait être utilisé non seulement pour traiter une infection virale mais également en prophylaxie, pour prévenir une infection virale.

**B-6. Q-VD-OPh inhibe l'expression d'un ensemble de protéines du VIH-1 dans les cellules** T **$CD4^+$ primaires**

Les inventeurs ont analysé l'expression, au 6ème jour post-infection, des protéines du VIH-1 dans les fractions cytosoliques, les fractions membranaires solubles et les fractions membranaires insolubles de lymphocytes T CD4+ infectés par le Virus VIH-1 puis incubés en présence ou en absence de le composé Q-VD-OPh. Le profil d'expression obtenu (voir Figure 6) montre que l'inhibiteur Q-VD-OPh diminue de façon drastique l'ensemble des protéines du VIH exprimées dans le cytosol et dans les fractions membranaires des lymphocytes T CD4+ primaires. En revanche, la présence d'inhibiteurs ne provoque pas une compartimentalisation différente des protéines, ni une accumulation des proformes. Le Q-VD-OPh a donc un effet inhibiteur pour l'expression de l'ensemble du génome viral mais n'a pas d'effet sur le trafic intracellulaire des protéines et donc sur la maturation des protéines.

**B-7. Q-VD-OPh inhibe la réplication de souches primaires de VIH-1.**

La réplication virale a été analysée par cytométrie en flux chez des lymphocytes T CD4+ infectés avec le virus VIH-1 Lai ou avec le sérum d'un patient VIH+ chronique contenant une souche primaire à double tropisme X4/R5.

Dans le cas du virus VIH-1 Lai comme dans le cas du sérum d'un patient VIH+ chronique, on constate que lorsque les lymphocytes infectés par le virus ont été cultivés en présence de le composé Q-VD-OPh, la réplication virale est fortement inhibée (Figure 5A). 3,79% de cellules infectées traitées avec Q-VD-OPh contre 36,46% des cellules non traitées avec Q-VD-OPh dans le cas d'une infection par la souche LAI et 0,06% cellules infectées traitées avec Q-VD-OPh dans le cas d'une infection par la souche primaire.

De plus, une analyse quantitative de la mort cellulaire par mesure de la dépolarisation mitochondriale, et de la réplication virale par dosage ELISA de l'antigène viral p24 chez des lymphocytes T CD4+ infectés avec le sérum du patient VIH+ chronique montrent que le Q-VD-OPh provoque également une forte inhibition de la mort cellulaire (inhibition totale ou quasi-totale) et de la réplication virale (Figure 5B) sur un isolat primaire du rétrovirus VIH-1.

En outre, les inventeurs ont analysé la réplication de cinq isolats primaires VIH-1 provenant de patients VIH+, de tropisme R5, en absence ou en présence d'inhibiteur Q-VD-OPh ou de ddl, un inhibiteur de la transcriptase inverse. On constate que pour les cinq isolats primaires, le Q-VD-OPh et le ddl ont un effet similaire, à savoir, une forte inhibition de la réplication virale (voir Figure 5C). Les souches virales à tropisme R5 sont les plus communément rencontrées et ont la caractéristique d'émerger précocement lors de l'infection et de persister au cours de l'évolution de la maladie alors que les souches virales à tropismes X4 sont plutôt des souches tardives. La capacité Q-VD-OPh à inhiber la réplication virale plus particulièrement des souches virales à tropisme R5 rend cette molécule très intéressante d'un point de vue thérapeutique car elle pourrait être utilisée pour stopper une infection virale à un stade très précoce.

**B-8. Q-VD-OPh inhibe la réplication du virus SIVmac251 et la formation de syncytia.**

Afin d'évaluer le champ d'application des propriétés antivirales de l'inhibiteur Q-VD-OPh, les inventeurs ont analysé l'effet de Q-VD-OPh sur la réplication du virus SIVmac251 et sur la formation de syncytia (Figure 8). La lignée cellulaire CEMx174 a été infectée avec une forte dose de virus de la souche SIVmac251 (200 AID50), qui correspond à dix fois la concentration utilisée pour infecter les macaques. L'analyse de la production virale dans le surnageant de culture par RT-PCR aux 4ème et 5ème jours post-infection démontre que la réplication du virus SIVmac251 est inhibée en présence d'inhibiteur Q-VD-OPh, et ce, de façon dose dépendante. De plus, lorsqu'on administre une forte concentration d'inhibiteur (20μM), la réplication virale est inhibée de plus de 70%. Par conséquent, il semble que le Q-VD-OPh inhibe de façon similaire la réplication des virus VIH et SIV.

Par ailleurs, la formation de syncytia, caractéristique qui serait liée à une plus ou moins grande virulence, est également fortement inhibée sous l'effet de l'inhibieur Q-VD-OPh. Il faut cependant une concentration en Q-VD-OPh égale ou supérieure à 10 μM pour obtenir une diminution de 70% du nombre de syncytia 5 jours après l'infection et le traitement.

Ces résultats démontrent que Q-VD-OPh est un inhibiteur de la réplication virale à large spectre, qui permet de bloquer non seulement les virus VIH mais également les virus SIV.

**B-9. Q-VD-OPh agit en synergie avec l'AZT et l'Indinavir pour inhiber la réplication virale et la mort des lymphocytes T CD4$^+$.**

Les inventeurs ont testé l'hypothèse selon laquelle l'inhibiteur Q-VD-OPh pourrait avoir un effet synergique avec d'autres molécules antivirales utilisées dans la lutte anti-VIH, en particulier avec l'azidothymidine (AZT), un inhibiteur de la transcriptase inverse, et avec l'Indinavir, un inhibiteur de protéase. Des cellules T CD4+ primaires ont été infectées par un virus VIH-Lai puis stimulées avec de la concanavaline A et de l'IL-2. Les cellules ont ensuite été cultivées en présence ou en absence de Q-VD-OPh (0,1, 1 ou 10 μM), et en présence ou en absence d'AZT (0.1 μM) ou d'Indinavir (1 μM). Dans le cas où les drogues ont été ajoutées à la culture, elles l'ont été à 96 heures (J3) après infection. La mort cellulaire a ensuite été quantifiée par cytométrie en flux au 5ème jour post-infection.

Les résultats obtenus (voir Figure 9) démontrent que le Q-VD-OPh, lorsqu'il est utilisé en combinaison avec AZT ou l'Indinavir, inhibe beaucoup plus fortement la mort cellulaire résultant de l'infection virale que le Q-VD-OPh, l'AZT ou l'Indinavir utilisés seuls. Par conséquent, Q-VD-OPh utilisé en association avec des traitements anti-VIH pour empêcher la mort cellulaire, conséquence de la réplication virale, donne lieu à un effet synergique.

**C. CONCLUSION**

L'ensemble de ces travaux démontre sans ambiguïté que le composé Q-VD-OPh est un puissant inhibiteur de la réplication des virus VIH et SIV. L'effet inhibiteur plus efficace du Q-VD-OPh administré avant l'infection (prétraitement - cf. exemple B5) montre de plus que cette molécule agit lors des premières étapes du cycle de réplication du virus. Le composé Q-VD-OPh revêt donc un intérêt majeur pour une utilisation thérapeutique en tant qu'agent antiviral et en particulier en tant qu'agent antirétroviral, et en tant qu'agent antilentiviral.

En outre, la capacité de Q-VD-OPh à prévenir l'apoptose pourrait être un atout supplémentaire permettant de restaurer de manière plus conséquente la réponse immune vis-à-vis des agents pathogènes.

Il est à noter que l'utilisation d'un autre inhibiteur à large spectre des caspases z-VAD-fmk n'inhibe pas l'apoptose de ces mêmes cellules au cours de la réplication virale (Petit et al, 2002)/ n'inhibe ni la réplication virale du VIH, ni la mort des lymphocytes T induite par l'infection par le VIH (Petit et al, 2002)0, suggérant un rôle majeur de ce nouvel inhibiteur dans la lutte contre cette infection virale.

Par ailleurs, Q-VD-OPh est capable d'agir en synergie avec d'autres molécules antivirales telles que l'AZT et l'Indinavir. L'utilisation de Q-VD-OPh en association avec d'autres molécules antivirales, en particulier avec d'autres molécules de l'arsenal anti-VIH actuellement disponible apparaît donc particulièrement prometteuse.

**EXEMPLE 2 : ANALYSE COMPARATIVE**

**A. MATERIEL ET METHODES**

***<u>Analyse par cvtofluorométrie de flux de la chute de $\lambda\varphi$m et de la protéine p24 du virus VIH</u>***

Pour évaluer le changement du potentiel transmembranaire de la membrane interne mitochondriale, les cellules ont été marquées par une sonde fluorescente $DIOC_6$ (Molecular Probes, Invitrogen), à une concentration de 40 nM et incubées pour 15 min à 37°C. La réplication virale a été évaluée par marquage interne de la protéine virale p24 utilisant

un anticorps anti-p24-PE (KC-57, Coulter Corp, Beckman).

***Immunoempreinte.***

Des extraits de 20 μg de lysat total préparés avec 1% NP40 ont été bouillis pour 5 min dans du tampon Laemmli contenant 2% SDS et 10% 2-β-mercaptoéthanol, puis déposés sur des gels de gradients de polyacrylamide de 10-20% (Invitrogen). Après transfert des protéines, les immunoempreintes ont été incubées avec les anticorps primaires suivant : anti-caspase-3, anti-caspase-8 et anti-caspase-9 (Cell Signaling), anti-PARP (Pharmingen) et anti-Tubuline (Santa-Cruz). Les anticorps secondaires couplés à la peroxidase (horseradish) (Amersham Biosciences) permet de révéler les protéines par chemiluminescence (ECL, Amersham) utilisant une caméra CCD (G :Box-Chemi-XT16 - SynGene).

***Inhibiteurs synthétiques et autres produits chimiques.***

L'inhibiteur de caspases à large spectre, Z-VAD-fmk (Calbiochem), l'inhibiteur général de caspases : Q-VD-OPH, l'inhibiteur de la caspase-8 : Q-IETD-OPH, l'ihnibiteur de la caspase-3,-7 :Q-DEVD-OPH et l'inhibiteur de la caspase-9 : Q-LEHD-OPH (MP Biomédicals, France) ont été utilisés. L'anticorps anti-CD95 (Fas Human) (clone 7C11, Immunotech) a été utilisé pour induire l'apoptose. Les anti-protéases du VIH, le Saquinavir, le Ritonavir et le Indinavir sont obtenus du NIH. La sonde CFSE (carboxyfluorecsein diacetate succinildyl ester) utilisé pour l'étude de la prolifération est obtenue de chez Molecular Probes(Invitrogen).

***Test de proliferation au CFSE***

Les PBMC sont incubés en présence de 1 μM de CFSE pendant 7min à 37 °C. Les cellules sont reprises à 1 .$10^6$/ml, puis mises en culture et activées par un anti-CD3 à 1 μg/ml (Immunotech).

**B. RESULTATS**

**B-1. Validation de nouveaux inhibiteurs de caspases**

***B-1-a. Détermination de la fonctionnalité de nouveaux inhibiteurs spécifiques des caspases -3, -8 et -9 vis-à-vis de l'apoptose induite par Fas/CD95.***

Des cellules Jurkat ont été incubées en présence ou non d'un anti-CD95 à différentes concentrations pour déterminer une courbe dose-réponse d'apoptose. La mort a été évaluée par analyse de la chute du potentiel transmembranaire mitochondriale (% Δφm low) en utilisant la sonde DIOC6 par cytométrie de flux (Figure 10A). La même expérience a été ensuite réalisée en présence de 0.25 μg/ml d'anti-CD95 et des différents inhibiteurs de caspases. L'apoptose induite par anti-CD95 est exprimée par le pourcentage du Δφm low analysé par cytométrie de flux (Figure 10B).

Des extraits de cellules Jurkat qui ont été traitées en présence ou non d'anti-CD95 et des différents inhibiteurs de caspases ont été analysés par immunoempreinte pour la caspase-3,-8,-9 et PARP (substrat spécifique de la caspase-3 et -7), la tubuline est utilisée comme contrôle des dépôts (Figure 10C).

***B-1-b. Détermination de la capacité des inhibiteurs spécifiques à inhiber la réplication virale et par conséquent la mort cellulaire.***

Des cellules T CD4 primaires ont été infectées par la souche virale VIH-Lai puis stimulées par la ConA/IL-2 en présence ou non du Q-VD-OPH, Q-DEVD-OPH (casp-3 inhibiteur), Q-LEHD-OPH (caspase-9 inhibiteur) et Q-IETD-OPH (caspase-8 inhibiteur) à 10 μM pour chacun des inhibiteurs. Au jour 5 et 7 post-infection, la protéine virale p24 interne est mesurée par cytométrie de flux après fixation et perméabilisation des cellules T CD4. Les résultats montrent un faible effet sur l'inhibition de la réplication virale pour chacun des différents inhibiteurs spécifiques des caspases à J5 et une absence de protection à J7, en revanche Q-VD-OPH inhibe complètement la réplication (Figure 11A). La mort a été évaluée par analyse de la chute du potentiel transmembranaire mitochondriale (% Δφm low) utilisant la sonde DIOC6 par cytométrie de flux (Figure 11B). Nous montrons une absence d'effet de ces inhibiteurs contrairement à Q-VD-OPH. Des extraits de cellules primaires T CD4 infectées en présence ou non des différents inhibiteurs de caspases ainsi que le QVD-OPH ont été analysés par immunoempreinte pour les protéines virales du VIH (Figure 12). Les résultats montrent que la quantité de protéines virales produites en présence des différents inhibiteurs est légèrement inférieure à celle des cultures contrôles mais que la production virale est très nettement diminuée en présence de QVD-OPH. Ces résultats sont en accord avec nos observations quant à la détection de la protéine p24 par cytométrie en flux.

**B-2. Effets toxiques des drogues**

***B-2-a. Le QVD-OPH à 50 μM ne bloque pas la prolifération des lymphocytes stimulés par 1μg/ml d'anti-CD-3.***

Une étude de prolifération lymphocytaire a été réalisée avec la sonde fluorescente CFSE qui permet de suivre la division cellulaire. Les PBMC sont stimulés par l'anti-CD3 à 1 μg/ml et incubés en présence ou en absence des anti-protéases du VIH, le Saquinavir, le Ritonavir et le Indinavir, à des concentrations de 1μM, 10μM et 50μM, ainsi que le QVD-OPH à 10μM, 50μM et 100μM. L'analyse du CFSE réalisée après 4 et 5 jours de stimulation montre par cytométrie de flux que les anti-protéases du VIH bloquent la prolifération de lymphocytes à la dose de 50 μM. En revanche, QVD-OPH, à ces mêmes concentrations n'a pas d'effet sur la prolifération des lymphocytes (Figure 13).

La toxicité cellulaire a été évaluée par l'analyse de la chute du potentiel transmembranaire mitochondriale (% Δφm low) utilisant la sonde DIOC6 par cytométrie de flux. Les cellules stimulées par anti-CD3 ont été analysées de la même façon au jour après activation. Ainsi, QVD-OPH, même à des concentrations élevées (100 μM) n'a pas d'effet sur la dépolarisation mitochondriale (par rapport au control) alors qu'Indinavir et Saquinavir montrent une chute de Δφm de plus de 70-80 % à la dose de 50 μM (Figure 14).

***B-2-b. Evaluation de la toxicité du QVD-OPH vis-à-vis des lymphocytes et des monocytes.***

Des PBMC sont incubés en présence des différents anti-protéases du VIH, le Saquinavir, le Ritonavir et le Indinavir, à des concentrations de 1μM, 10μM et 50μM, ainsi que le QVD-OPH à 10μM, 50μM et 100μM. La toxicité cellulaire a été évaluée par l'analyse de la chute du potentiel transmembranaire mitochondriale (% Δφm low) après 4 jours de culture en analysant les monocytes et les lymphocytes. L'effet du QVD-OPH sur la dépolarisation mitochondriale reste minime sur l'une ou l'autre des populations même à la concentration de 100 μM, tandis que les anti-protéases du VIH, en particulier le Saquinavir montre une dépolarisation de plus de 90 % (Figure 15A et B). Ces résultants sont en accord avec nos travaux antérieurs.

**BIBLIOGRAPHIE**

Alam et al. (1999). J. Exp. Med. 190(12): 1879-1890.
Amendola et al. (1996). Proc Natl Acad Sci USA. 93(20): 11057-62.
Barber (2001). Cell Death and Diff. 8: 113-126.
Caserta et al. (2003). Apoptosis. 8(4): 345-52.
Chinnaiyan et al. (1997). Nature Medicine. 3(3): 333-337.
Cryns et al. (1998). Genes & Development 12: 1551-1570.
Everett & McFadden. (1999). Trends in Microbiology. 7: 160-165.
Finkel et al. (1995). Nat Med. 1(2): 129-34.
Gandhi et al. (1998). The Journal of Experimental Medecine. 187(7): 1113-1122.
Gordon et al. (2005). The call of the wild: what can be learned from studies of SIV infection of natural hosts? In Leitner T, Foley B, Hahn B, Marx P, McCutchan F, Mellors J, Wolinsky S, and Korber B (eds.). HIV Sequence Compendium, 2005. Theoretical Biology and Biophysics Group, Los Alamos National Laboratory, NM, LA-UR 04-7420. 2-29
Gougeon et al. (1996), J Immunol. 156(9): 3509-20.
Hurtrel et al. (2005). Cell Death Differ. 12: 979.
Lavrik et al. (2005). The journal of Clinical Investigation. 115(10): 2665-2672.
Levine et al. (1996). Proc Nanti Acad Sci. 93: 4810.
Liang et al. (1998). J Virol. 72: 8586.
Olsen et al. (1996). J Virol. 70: 663.
Petit et al. (2002). J Biol Chem. 277: 1477.
Sticht et al. (2005). Nat Struct Mol Biol. 12: 671.
Ternois et al. (2005). Nat Struct Mol Biol. 12: 678.
Thornberry and Labzebnik (1998). Science. 281: 1312.
Vera et al. (2005). Biology of Reproduction. 72 : 516-522.
Wurzer et al. (2003). EMBO J. 22: 2717.

## Revendications

1. Composé Q-VD-OPh de structure N-(2(quinolyl)valyl-aspartyl-(2,6-difluorophénoxy)méthyl cétone pour son utilisation pour la prophylaxie et/ou le traitement d'une infection virale provoquée par un virus de l'immunodéficience humaine (VIH).

2. Composé pour son utilisation selon la revendication 1, dans lequel ledit VIH est le VIH-1.

3. Composé pour son utilisation selon l'une quelconque des revendications 1 à 2, dans lequel ladite infection virale est le syndrome d'immunodéficience acquise (SIDA).

4. Composé pour son utilisation selon la revendication 3, dans lequel ledit animal est un mammifère non humain.

5. Composition comprenant à titre de principe actif le composé selon l'une quelconque des revendications 1 à 4 et comprenant en outre un ou plusieurs véhicule(s), diluant(s) ou adjuvant(s) ou une de leurs associations, pour son utilisation pour la prophylaxie et/ou le traitement d'une infection virale provoquée par un virus de l'immunodéficience humaine (VIH).

6. Composé pour son utilisation selon l'une quelconque des revendications 1 à 4, ou composition pour son utilisation selon la revendication 5, caractérisé(e) en ce qu'il (elle) est administré(e) à un animal ou à un humain avant que ledit animal ou humain ne soit exposé audit virus et/ou pendant l'exposition au virus et/ou après l'exposition au virus, en particulier dans les 48 heures après que ledit animal ou humain a été exposé audit virus.

7. Composé pour son utilisation selon l'une quelconque des revendications 1 à 4 ou 6, ou composition pour son utilisation selon la revendication 5, caractérisé(e) en ce qu'on procède à plusieurs administrations successives dudit composé ou de ladite composition.

8. Composition antivirale comprenant ou consistant en :

   (i) au moins un composé Q-VD-OPh tel que défini dans la revendication 1; et
   (ii) au moins un autre agent antiviral,

   et éventuellement un ou plusieurs véhicule(s), diluant(s) ou adjuvants ou une de leurs associations, pour son utilisation pour la prophylaxie et/ou le traitement d'une infection virale provoquée par un virus de l'immunodéficience humaine (VIH).

9. Combinaison comprenant ou consistant en :

   (i) au moins un composé Q-VD-OPh tel que défini dans la revendication 1; et
   (ii) au moins un autre agent antiviral,

   et éventuellement un ou plusieurs véhicule(s), diluant(s) ou adjuvants ou une de leurs associations,
   dans laquelle les composés (i) et (ii) sont séparés l'un de l'autre, pour son utilisation pour la prophylaxie et/ou le traitement d'une infection virale provoquée par un virus de l'immunodéficience humaine (VIH).

10. Combinaison pour son utilisation selon la revendication 9 ou composition antivirale pour son utilisation selon la revendication 8, ladite utilisation étant simultanée, séparée ou étalée dans le temps.

11. Composition antivirale pour son utilisation selon la revendication 8 ou combinaison pour son utilisation selon l'une quelconque des revendications 9 à 10, dans laquelle ledit autre agent antiviral est choisi parmi :

    - les inhibiteurs de transcriptase;
    - les inhibiteurs de la protéase virale (ou antiprotéases);
    - les inhibiteurs de la fusion de l'enveloppe virale avec la membrane de la cellule;
    - des inhibiteurs de récepteurs ou co-récepteurs;
    - des oligonucléotides anti-sens ;
    - les inhibiteurs de l'intégrase ; et
    - des molécules ciblant d'autres étapes de la multiplication virale.

**12.** Composition antivirale ou combinaison pour son utilisation selon l'une quelconque des revendications 8 à 11, dans laquelle ledit autre agent antiviral ou au moins un desdits autres agents antiviraux consiste(nt) en au moins un inhibiteur de transcriptase et/ou au moins un inhibiteur de protéase virale,.

**13.** Composition antivirale ou combinaison pour son utilisation selon la revendication 12, **caractérisée en ce que** l'inhibiteur de transcriptase inverse est choisi parmi le groupe constitué de la zidovudine ou azidothymidine (AZT), la didanosine ou ddl, la zalcitabine ou ddC, la stavudine ou d4T, la lamivudine ou 3TC, l'abacavir ou ABC, et l'emtricitabine ou FTC, la nevirapine, l'efavirenz, la délavirdine et le ténofovir ou bis-POC-PMPA et/ou que l'inhibiteur de protéase virale est l'Indinavir.

**14.** Composition pour son utilisation selon la revendication 5, composition antivirale ou combinaison pour son utilisation selon l'une quelconque des revendications 8 à 13, dans laquelle les composés (i) et (ii) sont formulé(s) pour une administration par voie entérale, parentérale, transcutanée, cutanée, orale, ophtalmique, otologique, mucosale, ou encore les voies intragastrique, intracardiaque, intrapéritonéale, intrapulmonaire ou intratrachéale.

**15.** Composition antivirale ou combinaison pour son utilisation selon l'une quelconque des revendications 10 à 14, dans laquelle ledit animal est un mammifère non humain.

## Patentansprüche

**1.** Verbindung Q-VD-OPh der Struktur N-(2(quinolyl)valyl-aspartyl-(2,6-difluorophenoxy)-Methylketon zur Verwendung zur Vorsorge und/oder zur Behandlung einer Virusinfektion, die durch ein Human-Immundefizienz-Virus (HIV) bewirkt wird.

**2.** Verbindung zur Verwendung nach Anspruch 1, wobei das HIV das HIV-1 ist.

**3.** Verbindung zur Verwendung nach einem der Ansprüche 1 bis 2, wobei die Virusinfektion das erworbene Immundefizienzsyndrom (AIDS) ist.

**4.** Verbindung zur Verwendung nach Anspruch 3, wobei das Tier ein nicht-menschliches Säugetier ist.

**5.** Zusammensetzung, die als Wirkstoff die Verbindung nach einem der Ansprüche 1 bis 4 umfasst und außerdem ein oder mehrere Verdünnungsmittel oder Zusatzmittel oder eine ihrer Verbindungen umfasst, zur Verwendung zur Vorsorge und/oder zur Behandlung einer Virusinfektion, die durch ein Human-Immundefizienz-Virus (HIV) bewirkt wird.

**6.** Verbindung zur Verwendung nach einem der Ansprüche 1 bis 4 oder Zusammensetzung zur Verwendung nach Anspruch 5, **dadurch gekennzeichnet, dass** sie einem Tier oder einem Menschen verabreicht wird, bevor das Tier oder der Mensch dem Virus ausgesetzt ist und/oder während des Ausgesetzt-Seins gegenüber dem Virus und/oder nach dem Ausgesetzt-Sein gegenüber dem Virus, insbesondere innerhalb der 48 Stunden, nachdem das Tier oder der Mensch dem Virus ausgesetzt war.

**7.** Verbindung zur Verwendung nach einem der Ansprüche 1 bis 4 oder 6 oder Zusammensetzung zur Verwendung nach Anspruch 5, **dadurch gekennzeichnet, dass** mehrere aufeinanderfolgende Verabreichungen der Verbindung oder der Zusammensetzung durchgeführt werden.

**8.** Antivirale Zusammensetzung, die Folgendes umfasst oder aus Folgendem besteht:

(i) mindestens eine Verbindung Q-VD-OPh wie zuvor in Anspruch 1 definiert; und
(ii) mindestens ein weiteres antivirales Mittel,

und eventuell ein oder mehrere Verdünnungsmittel, Zusatzmittel oder eine ihrer Verbindungen zur Verwendung zur Vorsorge und/oder zur Behandlung einer Virusinfektion, die durch ein Human-Immundefizienz-Virus (HIV) bewirkt wird.

**9.** Kombination, die Folgendes umfasst oder aus Folgendem besteht:

(i) mindestens eine Verbindung Q-VD-OPh wie zuvor in Anspruch 1 definiert; und
(ii) mindestens ein weiteres antivirales Mittel,

und eventuell ein oder mehrere Verdünnungsmittel, Zusatzmittel oder eine ihrer Verbindungen, wobei die Verbindungen (i) und (ii) voneinander getrennt sind, zur Verwendung zur Vorsorge und/oder zur Behandlung einer Virusinfektion, die durch ein Human-Immundefizienz-Virus (HIV) bewirkt wird.

**10.** Kombination zur Verwendung nach Anspruch 9 oder antivirale Zusammensetzung zur Verwendung nach Anspruch 8, wobei deren Verwendung gleichzeitig, getrennt oder über die Zeit verteilt stattfindet.

**11.** Antivirale Zusammensetzung zur Verwendung nach Anspruch 8 oder Kombination zur Verwendung nach einem der Ansprüche 9 und 10, wobei das andere antivirale Mittel aus Folgendem gewählt ist:

- den Hemmstoffen der Transkriptase;
- den Hemmstoffen der Virusprotease (oder Antiproteasen);
- den Hemmstoffen der Verschmelzung der Virushülle mit der Zellmembran;
- Hemmstoffen der Rezeptoren oder Co-Rezeptoren;
- Antisense-Oligonukleotiden;
- den Hemmstoffen der Integrase; und
- Molekülen, die auf andere Phasen der viralen Vermehrung ausgerichtet sind.

**12.** Antivirale Zusammensetzung oder Kombination zur Verwendung nach einem der Ansprüche 8 bis 11, wobei das andere antivirale Mittel oder mindestens eines der anderen antiviralen Mittel mindestens aus einem Hemmstoff für die Transkriptase und/oder mindestens einem Hemmstoff für die Virusprotease besteht.

**13.** Antivirale Zusammensetzung oder Kombination zur Verwendung nach Anspruch 12, **dadurch gekennzeichnet, dass** der Hemmstoff der Reversen Transkriptase aus der Gruppe gewählt ist, die aus Zidovudin oder Azidothymidin (AZT), Didanosin oder ddl, Zalcitabin oder ddC, Stavudin oder d4T, Lamivudin oder 3TC, Abacavir oder ABC und Emtricitabin oder FTC, Nevirapin, Efavirenz, Delavirdin und Tenofovir oder bis-POC-PMPA besteht, und/oder dass der Hemmstoff der Virusprotease Indinavir ist.

**14.** Zusammensetzung zur Verwendung nach Anspruch 5, antivirale Zusammensetzung oder Kombination zur Verwendung nach einem der Ansprüche 8 bis 13, wobei die Verbindungen (i) und (ii) eine Formel/Formeln für eine Verabreichung enteral, parenteral, transkutan, kutan, oral, ophthalmisch, otologisch, mucosal oder außerdem für die Verabreichungswege intragastral, intrakardial, intraperitoneal, intrapulmonal oder intratracheal sind.

**15.** Antivirale Zusammensetzung oder Kombination zur Verwendung nach einem der Ansprüche 10 bis 14, wobei das Tier ein nicht-menschliches Säugetier ist.

## Claims

**1.** Compound Q-VD-OPh of the structure N-(2(quinolyl)valyl-aspartyl-(2,6-difluorophenoxy)methyl ketone for use in the prophylaxis and/or treatment of a viral infection caused by a human immunodeficiency virus (HIV).

**2.** Compound for use according to claim 1, wherein said HIV is HIV-1.

**3.** Compound for use according to either claim 1 or claim 2, wherein said viral infection is acquired immunodeficiency syndrome (AIDS).

**4.** Compound for use according to claim 3, wherein said animal is a non-human mammal.

**5.** Composition comprising as active ingredient the compound according to any one of claims 1 to 4 and further comprising one or more carrier(s), diluent(s) or adjuvant(s) or an association thereof, for use in the prophylaxis and/or treatment of a viral infection caused by a human immunodeficiency virus (HIV).

**6.** Compound for use according to any one of claims 1 to 4, or composition for use according to claim 5, **characterised in that** it is administered to an animal or to a human before said animal or human is exposed to said virus and/or

during exposure to the virus and/or after exposure to the virus, especially in the 48 hours after said animal or human has been exposed to said virus.

7. Compound for use according to any one of claims 1 to 4 or 6, or composition for use according to claim 5, **characterised in that** a plurality of successive administrations of said compound or of said composition are carried out.

8. Antiviral composition comprising or consisting of:

(i) at least one compound Q-VD-OPh as defined in claim 1; and
(ii) at least one other antiviral agent,

and optionally one or more carrier(s), diluent(s) or adjuvant(s) or an association thereof, for use in the prophylaxis and/or treatment of a viral infection caused by a human immunodeficiency virus (HIV).

9. Combination comprising or consisting of:

(i) at least one compound Q-VD-OPh as defined in claim 1; and
(ii) at least one other antiviral agent,

and optionally one or more carrier(s), diluent(s) or adjuvant(s) or an association thereof,
wherein the compounds (i) and (ii) are separate from one another, for use in the prophylaxis and/or treatment of a viral infection caused by a human immunodeficiency virus (HIV).

10. Combination for use according to claim 9 or antiviral composition for use according to claim 8, said use being simultaneous, separate or spread over time.

11. Antiviral composition for use according to claim 8 or combination for use according to either claim 9 or claim 10, wherein said other antiviral agent is chosen from:

- transcriptase inhibitors;
- viral protease inhibitors (or antiproteases);
- inhibitors of the fusion of the viral envelope with the cell membrane;
- receptor or co-receptor inhibitors;
- anti-sense oligonucleotides;
- integrase inhibitors; and
- molecules targeting other steps of viral multiplication.

12. Antiviral composition or combination for use according to any one of claims 8 to 11, wherein said other antiviral agent or at least one of said other antiviral agents consist(s) of at least one transcriptase inhibitor and/or at least one viral protease inhibitor.

13. Antiviral composition or combination for use according to claim 12, **characterised in that** the reverse-transcriptase inhibitor is chosen from the group consisting of zidovudine or azidothymidine (AZT), didanosine or ddl, zalcitabine or ddC, stavudine or d4T, lamivudine or 3TC, abacavir or ABC, and emtricitabine or FTC, nevirapine, efavirenz, delavirdine and tenofovir or bis-POC-PMPA and/or **in that** the viral protease inhibitor is indinavir.

14. Composition for use according to claim 5, antiviral composition or combination for use according to any one of claims 8 to 13, wherein compounds (i) and (ii) are formulated for administration by the enteral, parenteral, transcutaneous, cutaneous, oral, ophthalmic, otological or mucosal route or by the intragastric, intracardiac, intraperitoneal, intrapulmonary or intratracheal routes.

15. Antiviral composition or combination for use according to any one of claims 10 to 14, wherein said animal is a non-human mammal.

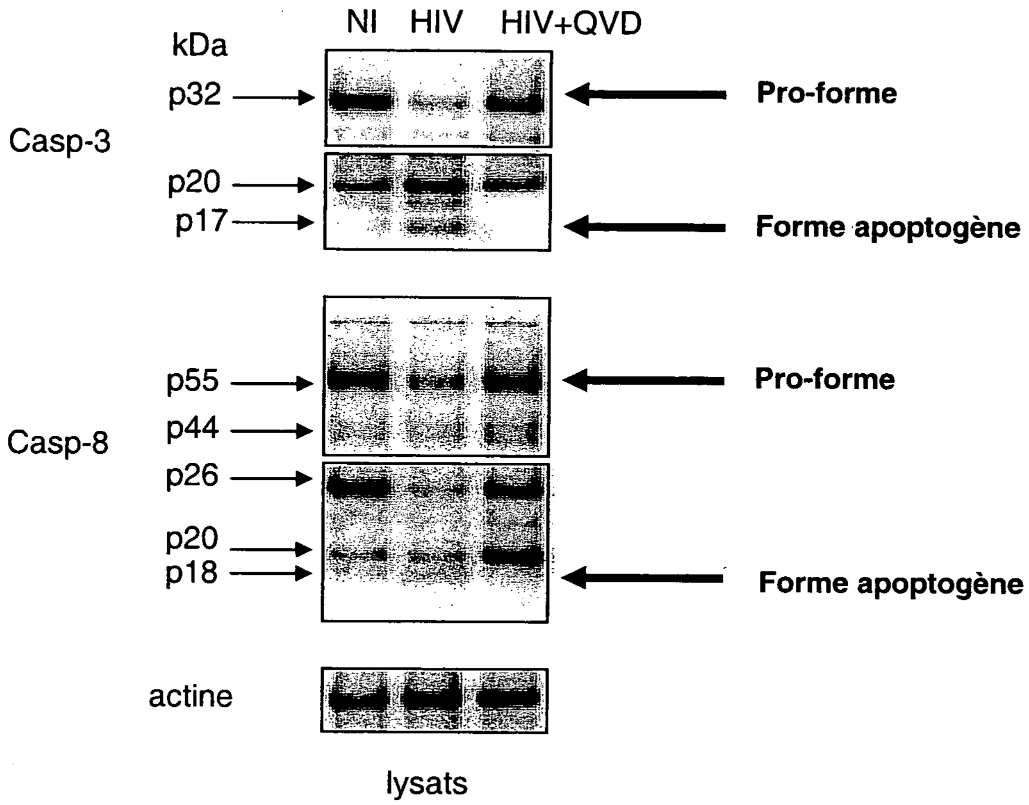
NI HIV HIV+QVD
kDa
p32
Pro-forme
Casp-3
p20
p17
Forme apoptogène
p55
Pro-forme
p44
Casp-8
p26
p20
p18
Forme apoptogène
actine
lysats

# Figure 1

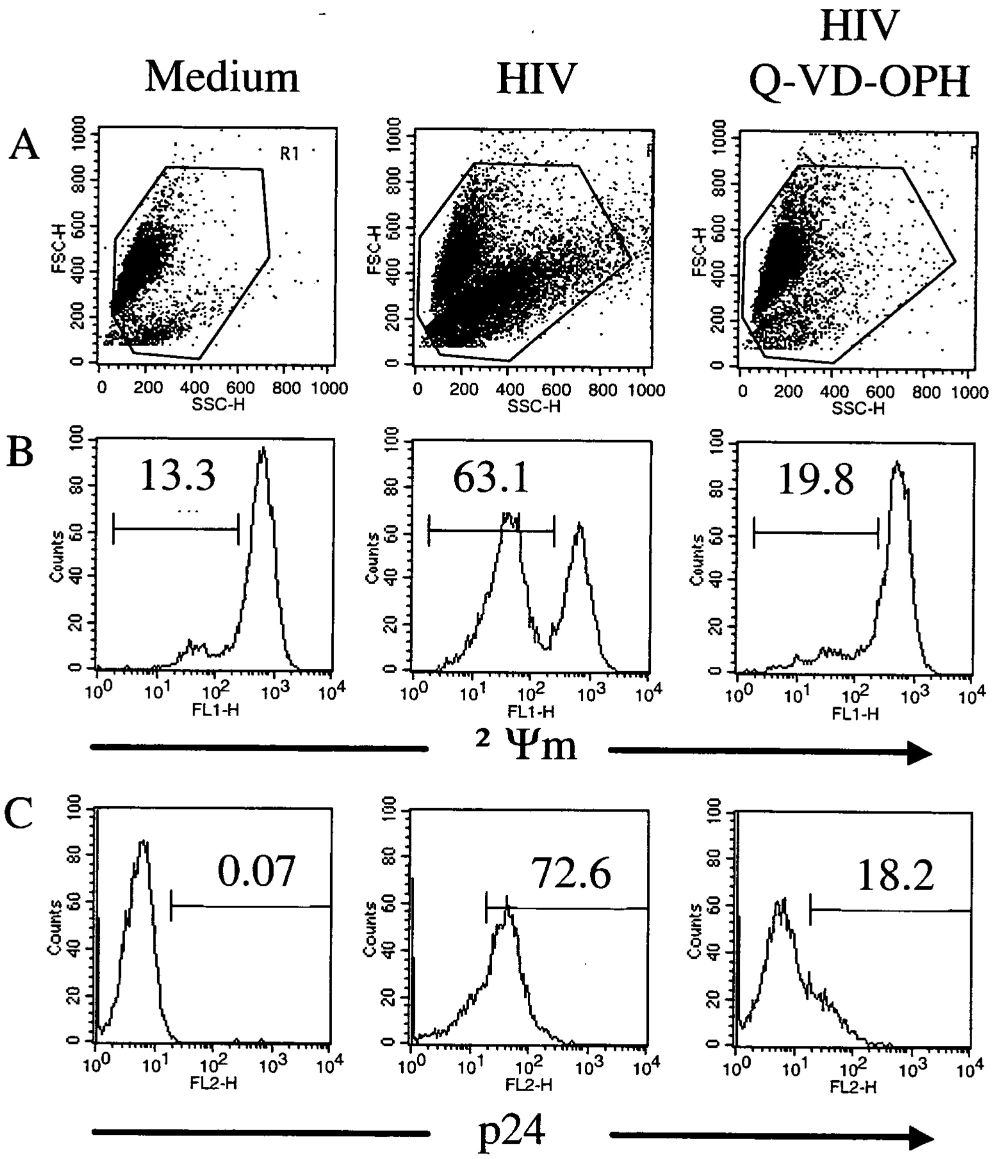
Medium
HIV
HIV
Q-VD-OPH
A
B
C
R1
FSC-H
SSC-H
Counts
FL1-H
FL2-H
13.3
63.1
19.8
0.07
72.6
18.2
² Ψm
p24

Figure 2

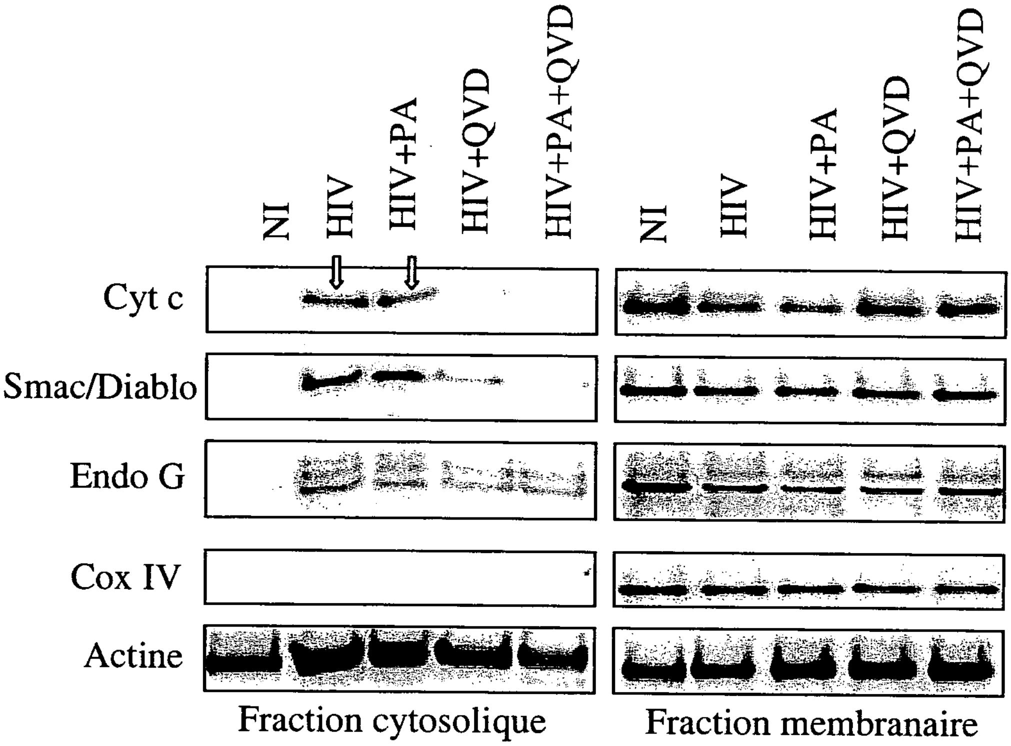
NI
HIV
HIV+PA
HIV+QVD
HIV+PA+QVD
NI
HIV
HIV+PA
HIV+QVD
HIV+PA+QVD
Cyt c
Smac/Diablo
Endo G
Cox IV
Actine
Fraction cytosolique
Fraction membranaire

Figure 3

A

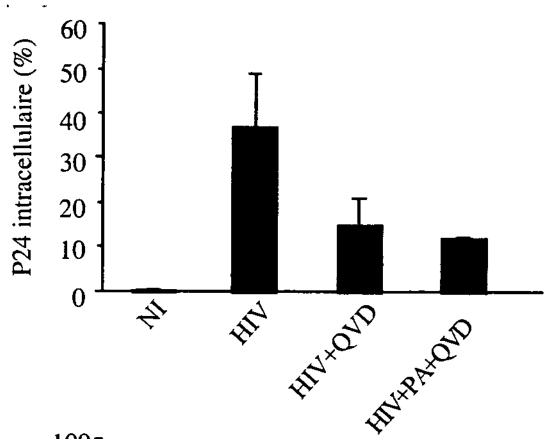
P24 intracellulaire (%)
60
50
40
30
20
10
0
NI
HIV
HIV+QVD
HIV+PA+QVD

B

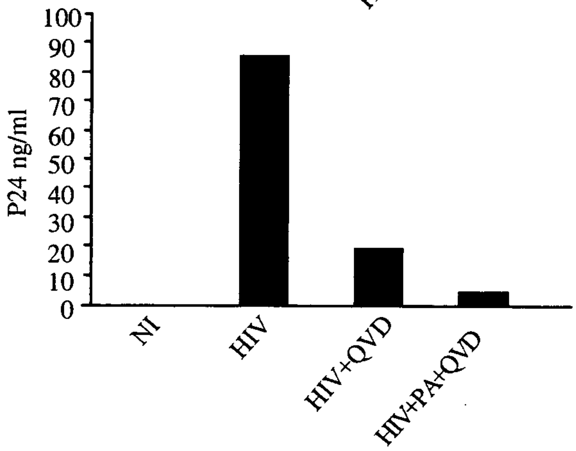
P24 ng/ml
100
90
80
70
60
50
40
30
20
10
0
NI
HIV
HIV+QVD
HIV+PA+QVD

**Figure 4**

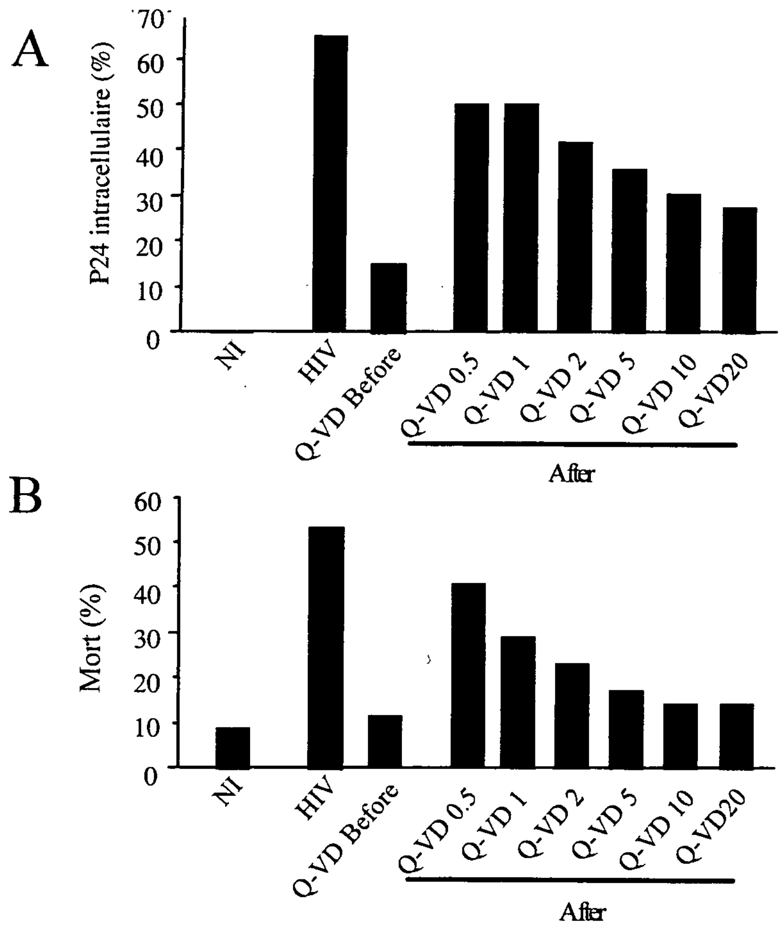
A
P24 intracellulaire (%)
70
60
50
40
30
20
10
0
NI
HIV
Q-VD Before
Q-VD 0.5
Q-VD 1
Q-VD 2
Q-VD 5
Q-VD 10
Q-VD20
After
B
Mort (%)
60
50
40
30
20
10
0
NI
HIV
Q-VD Before
Q-VD 0.5
Q-VD 1
Q-VD 2
Q-VD 5
Q-VD 10
Q-VD20
After

**Figure 5**

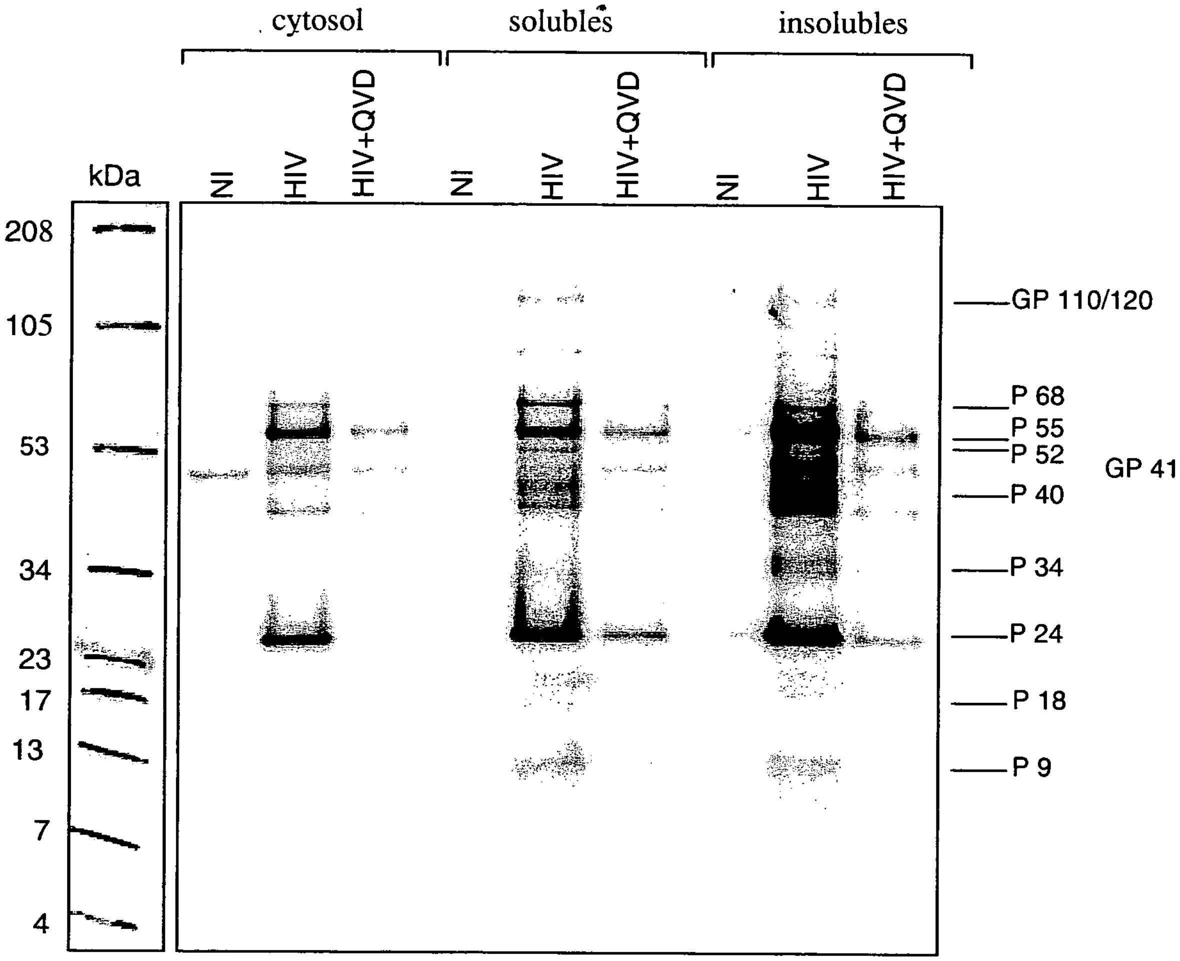
cytosol
solubles
insolubles
kDa
NI
HIV
HIV+QVD
NI
HIV
HIV+QVD
NI
HIV
HIV+QVD
208
105
53
34
23
17
13
7
4
GP 110/120
P 68
P 55
P 52
GP 41
P 40
P 34
P 24
P 18
P 9

Figure 6

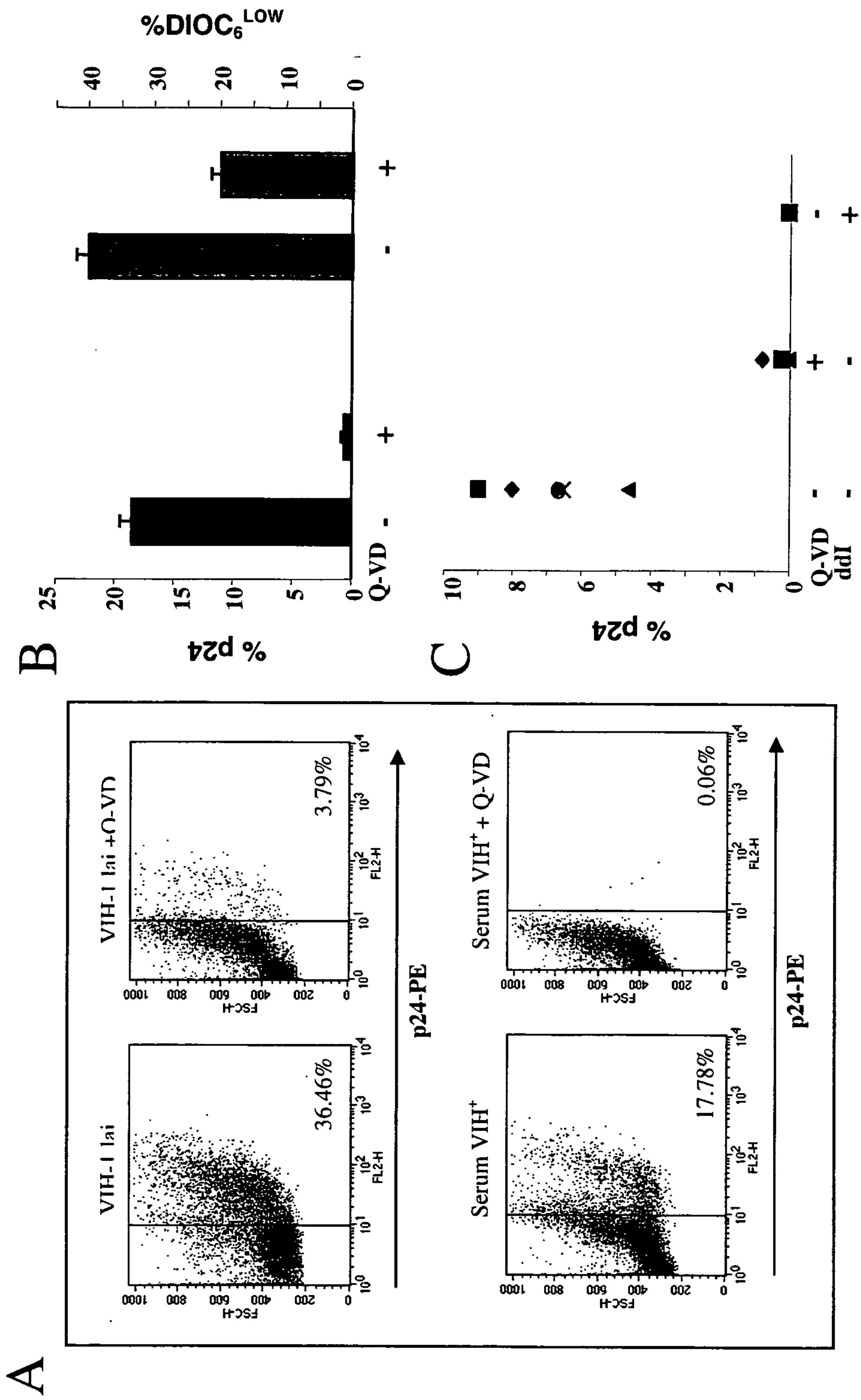
A
VIH-1 lai
36.46%
VIH-1 lai +Q-VD
3.79%
Serum VIH+
17.78%
Serum VIH+ + Q-VD
0.06%
FSC-H
FL2-H
p24-PE
B
% p24
%DIOC6 LOW
Q-VD
C
% p24
Q-VD
ddI

Figure 7

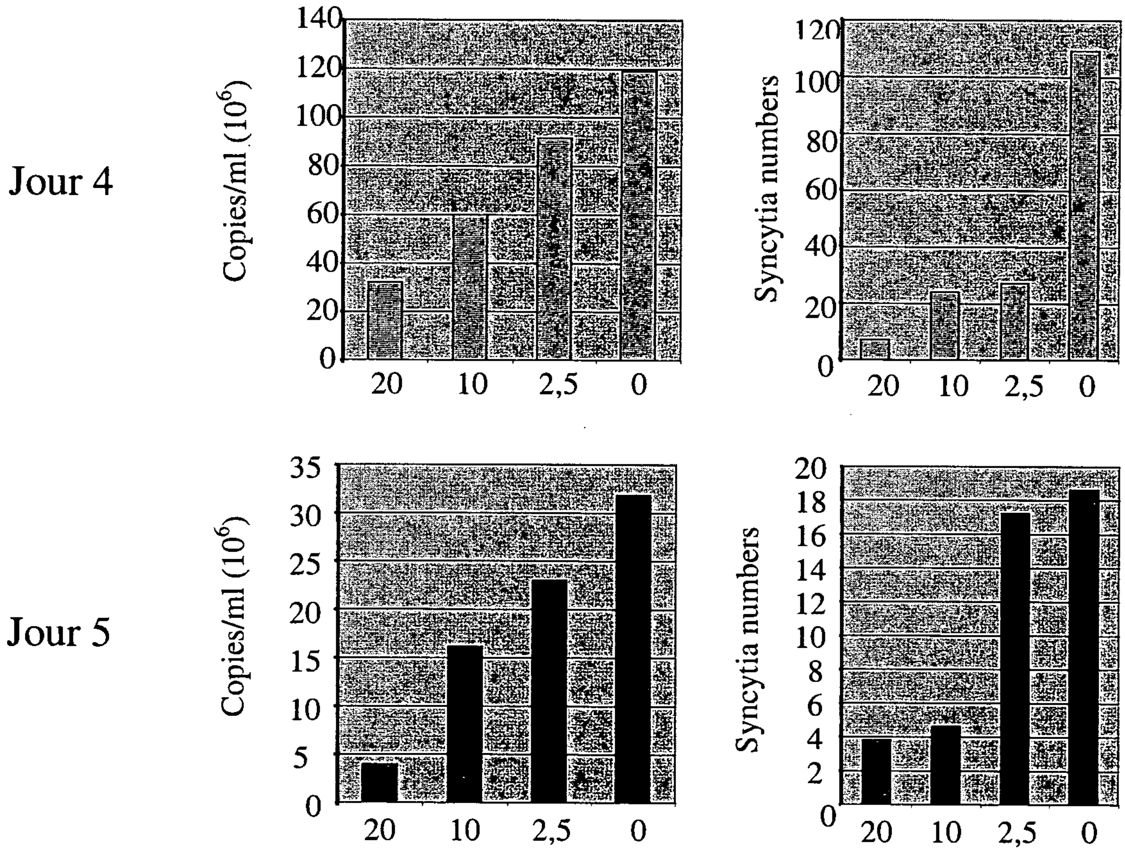
Jour 4
Copies/ml (10^6)
140
120
100
80
60
40
20
0
20
10
2,5
0
Syncytia numbers
120
100
80
60
40
20
0
20
10
2,5
0
Jour 5
Copies/ml (10^6)
35
30
25
20
15
10
5
0
20
10
2,5
0
Syncytia numbers
20
18
16
14
12
10
8
6
4
2
0
20
10
2,5
0

**Figure 8**

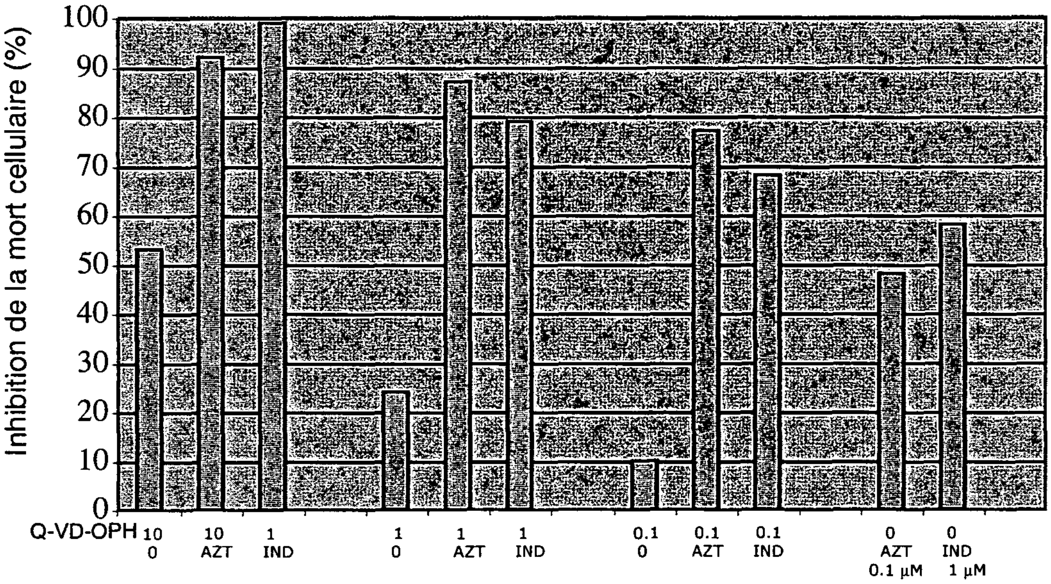
Inhibition de la mort cellulaire (%)
100
90
80
70
60
50
40
30
20
10
0
Q-VD-OPH 10 0
10 AZT
1 IND
1 0
1 AZT
1 IND
0.1 0
0.1 AZT
0.1 IND
0 AZT 0.1 µM
0 IND 1 µM

# Figure 9

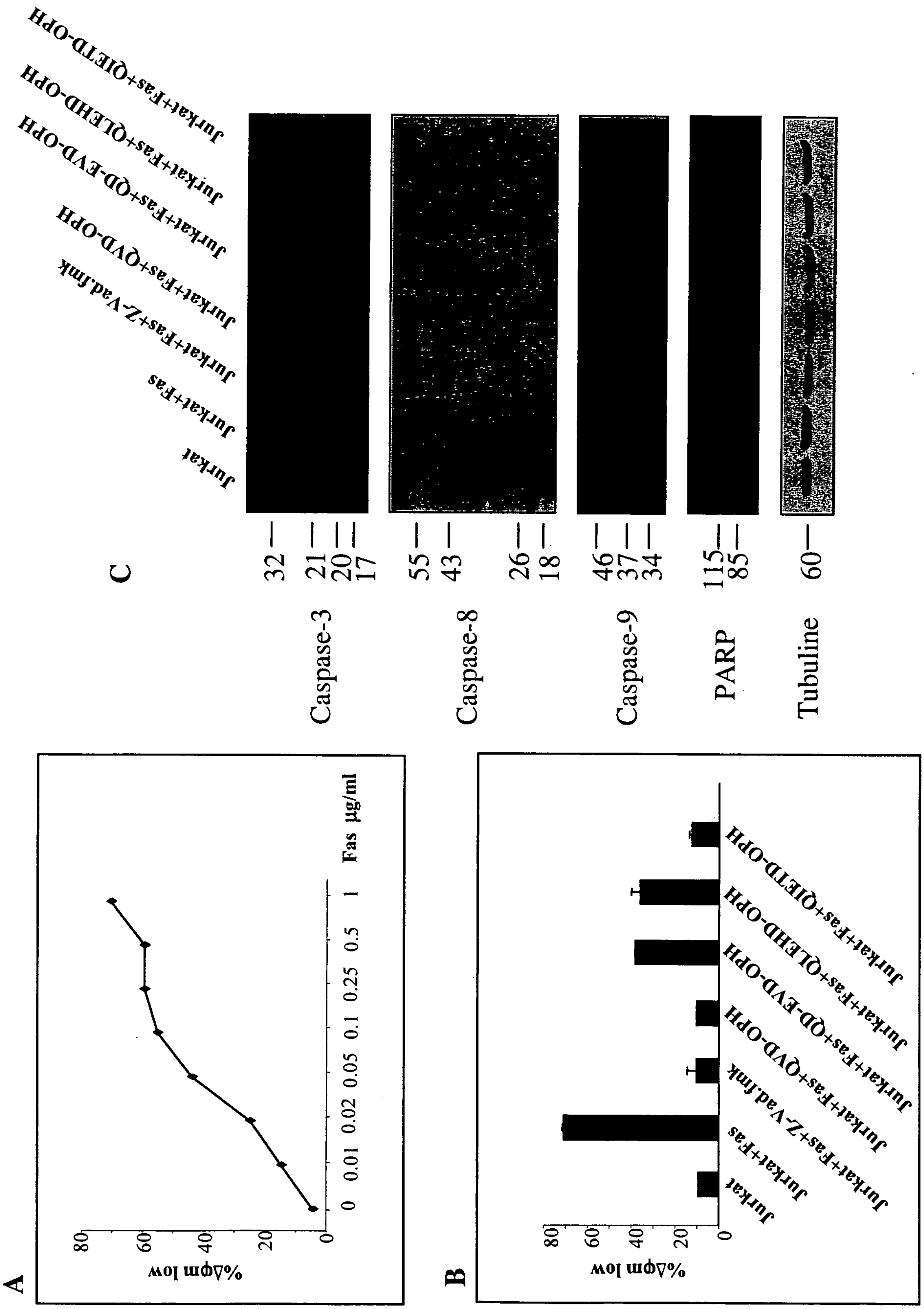
A
%Δφm low
0
20
40
60
80
0
0.01
0.02
0.05
0.1
0.25
0.5
1
Fas µg/ml
B
%Δφm low
0
20
40
60
80
Jurkat
Jurkat+Fas
Jurkat+Fas+Z-Vad.fmk
Jurkat+Fas+QVD-OPH
Jurkat+Fas+QD-EVD-OPH
Jurkat+Fas+QLEHD-OPH
Jurkat+Fas+QIETD-OPH
C
Jurkat
Jurkat+Fas
Jurkat+Fas+Z-Vad.fmk
Jurkat+Fas+QVD-OPH
Jurkat+Fas+QD-EVD-OPH
Jurkat+Fas+QLEHD-OPH
Jurkat+Fas+QIETD-OPH
Caspase-3
32
21
20
17
Caspase-8
55
43
26
18
Caspase-9
46
37
34
PARP
115
85
Tubuline
60

Figure 10

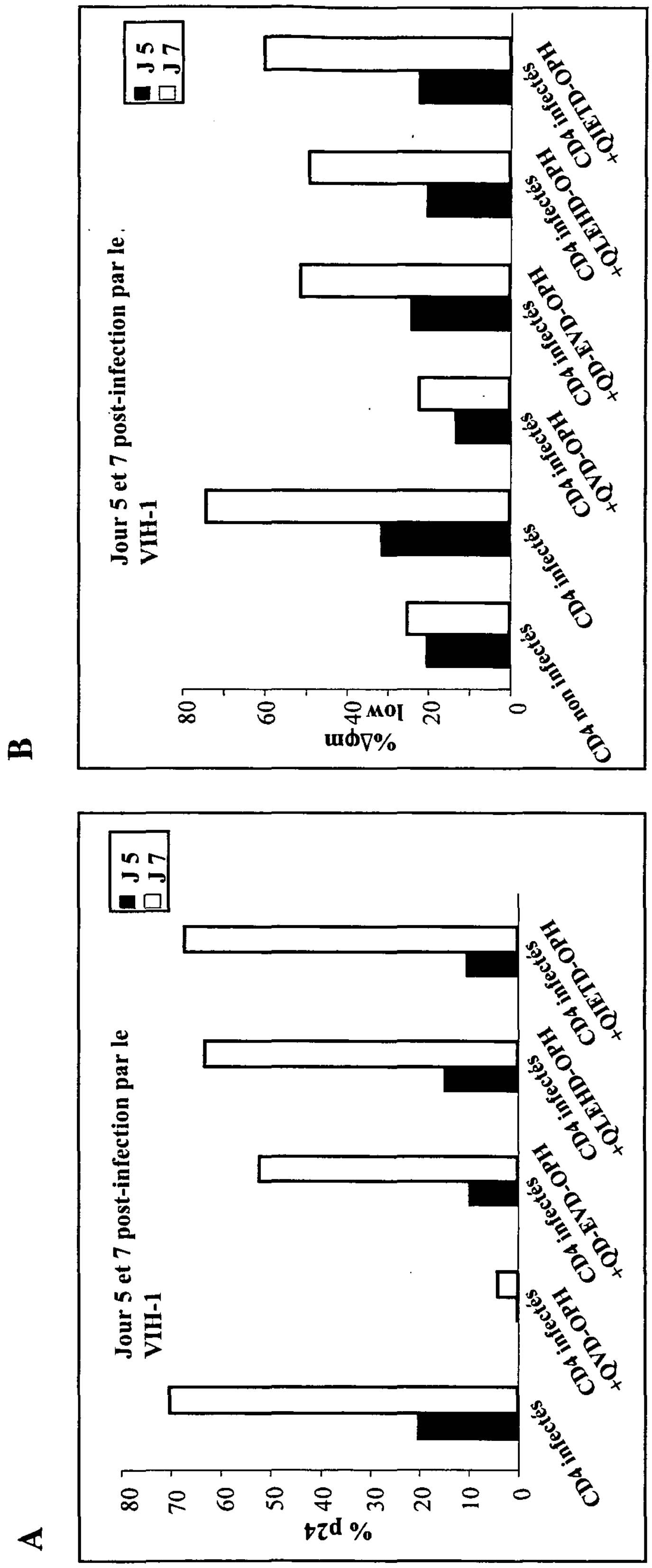
B
Jour 5 et 7 post-infection par le VIH-1
J 5
J 7
%Δφm low
0
20
40
60
80
CD4 non infectés
CD4 infectés
CD4 infectés +QVD-OPH
CD4 infectés +QD-EVD-OPH
CD4 infectés +QLEHD-OPH
CD4 infectés +QIETD-OPH
A
Jour 5 et 7 post-infection par le VIH-1
J 5
J 7
% p24
0
10
20
30
40
50
60
70
80
CD4 infectés
CD4 infectés +QVD-OPH
CD4 infectés +QD-EVD-OPH
CD4 infectés +QLEHD-OPH
CD4 infectés +QIETD-OPH

Figure 11

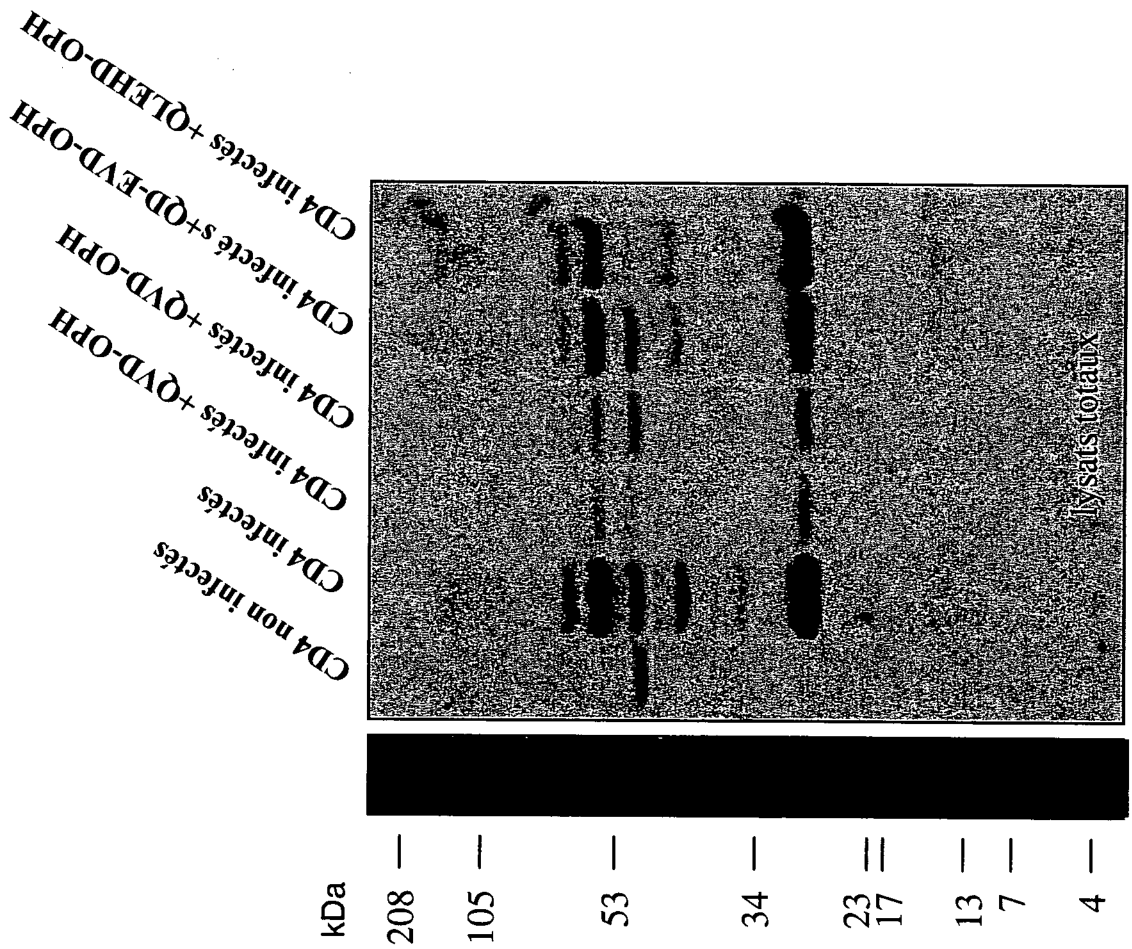
CD4 infectés +QLEHD-OPH
CD4 infecté s+QD-EVD-OPH
CD4 infectés +QVD-OPH
CD4 infectés +QVD-OPH
CD4 infectés
CD4 non infectés
Lysats totaux
kDa
208
105
53
34
23
17
13
7
4

Figure 12

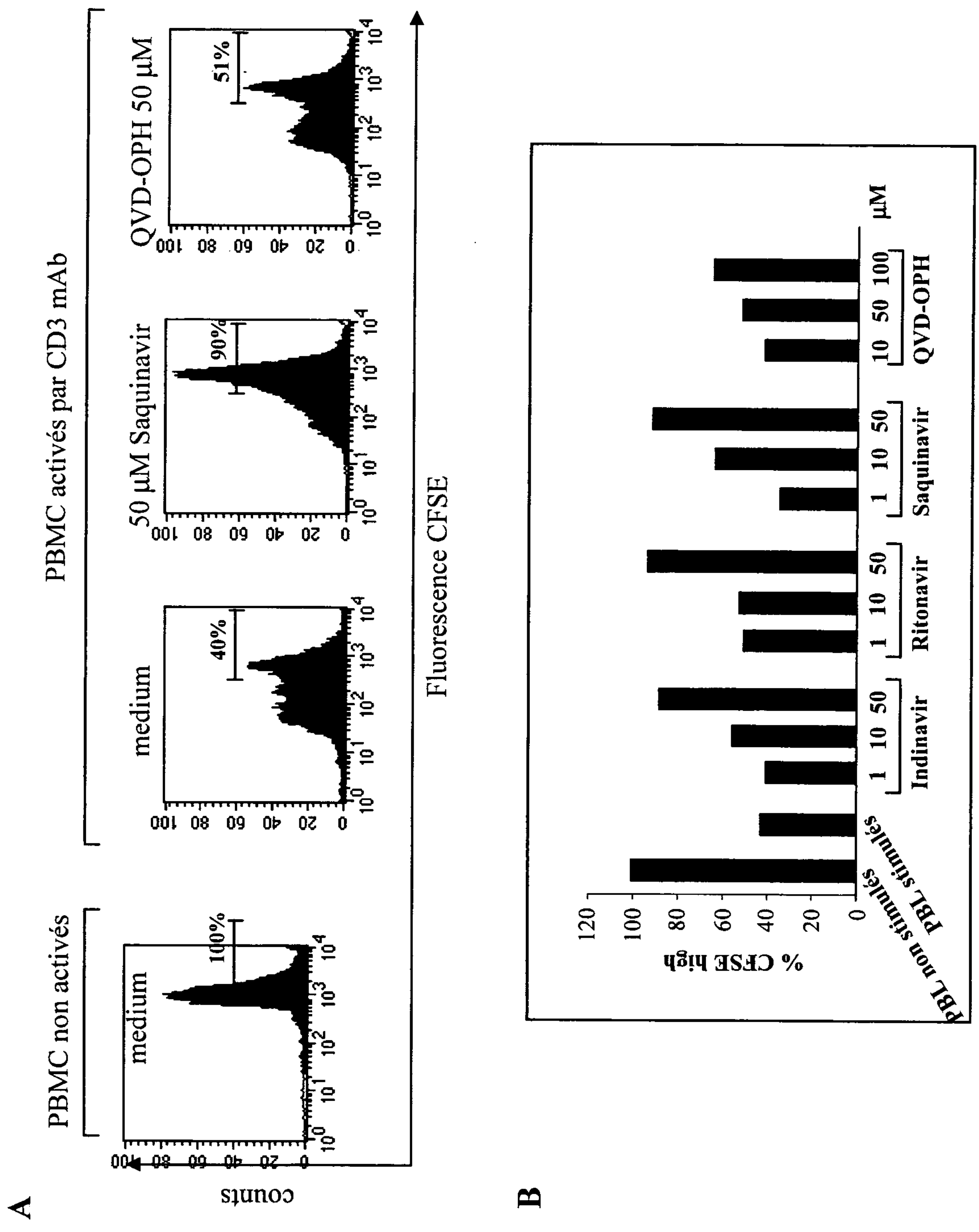
A
B
PBMC non activés
PBMC activés par CD3 mAb
medium
medium
50 µM Saquinavir
QVD-OPH 50 µM
100%
40%
90%
51%
counts
Fluorescence CFSE
% CFSE high
PBL non stimulés
PBL stimulés
Indinavir
Ritonavir
Saquinavir
QVD-OPH
µM

Figure 13

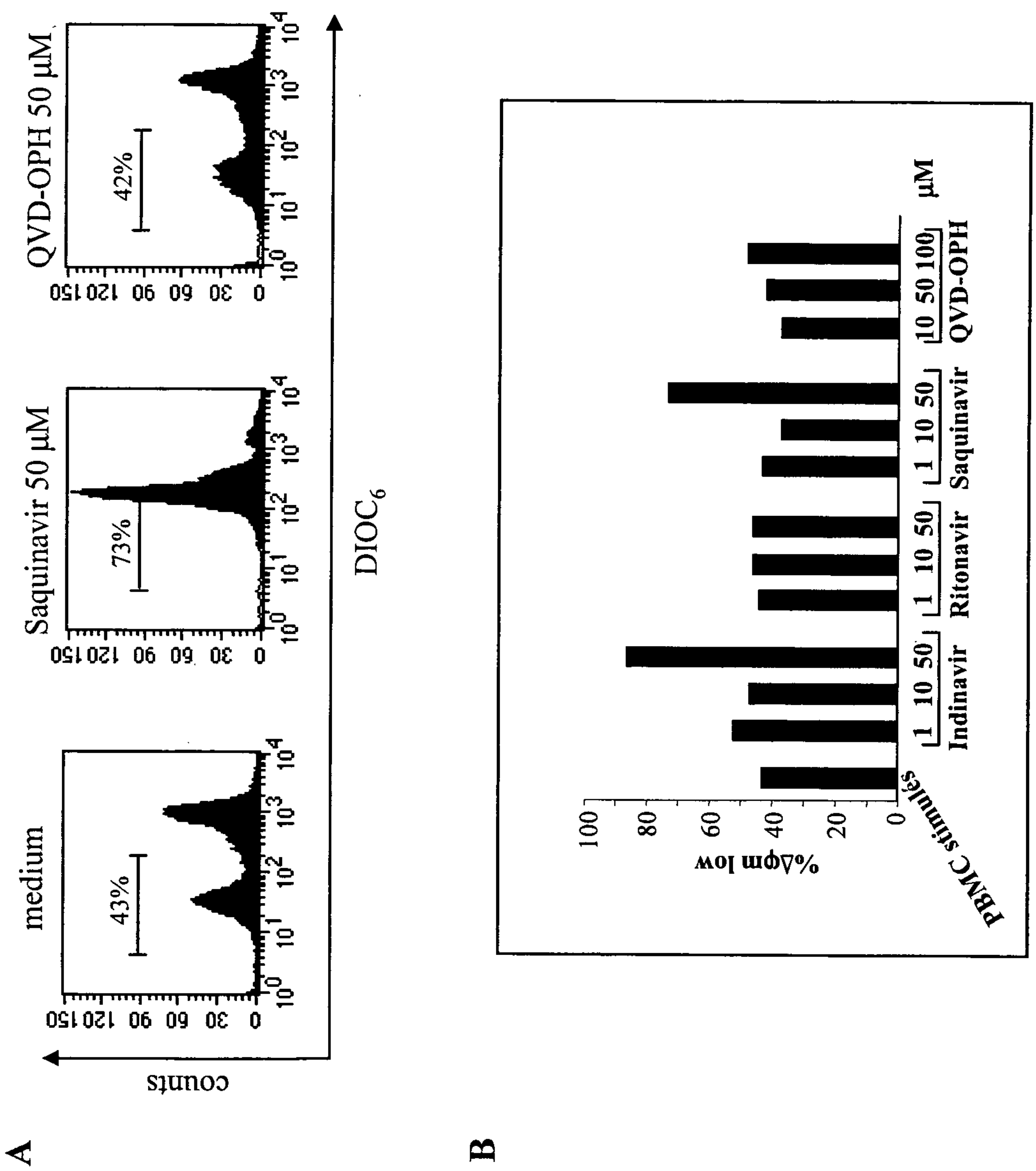
A
medium
Saquinavir 50 µM
QVD-OPH 50 µM
43%
73%
42%
counts
DIOC$_6$
B
%Δφm low
PBMC stimulés
Indinavir
Ritonavir
Saquinavir
QVD-OPH
µM

Figure 14

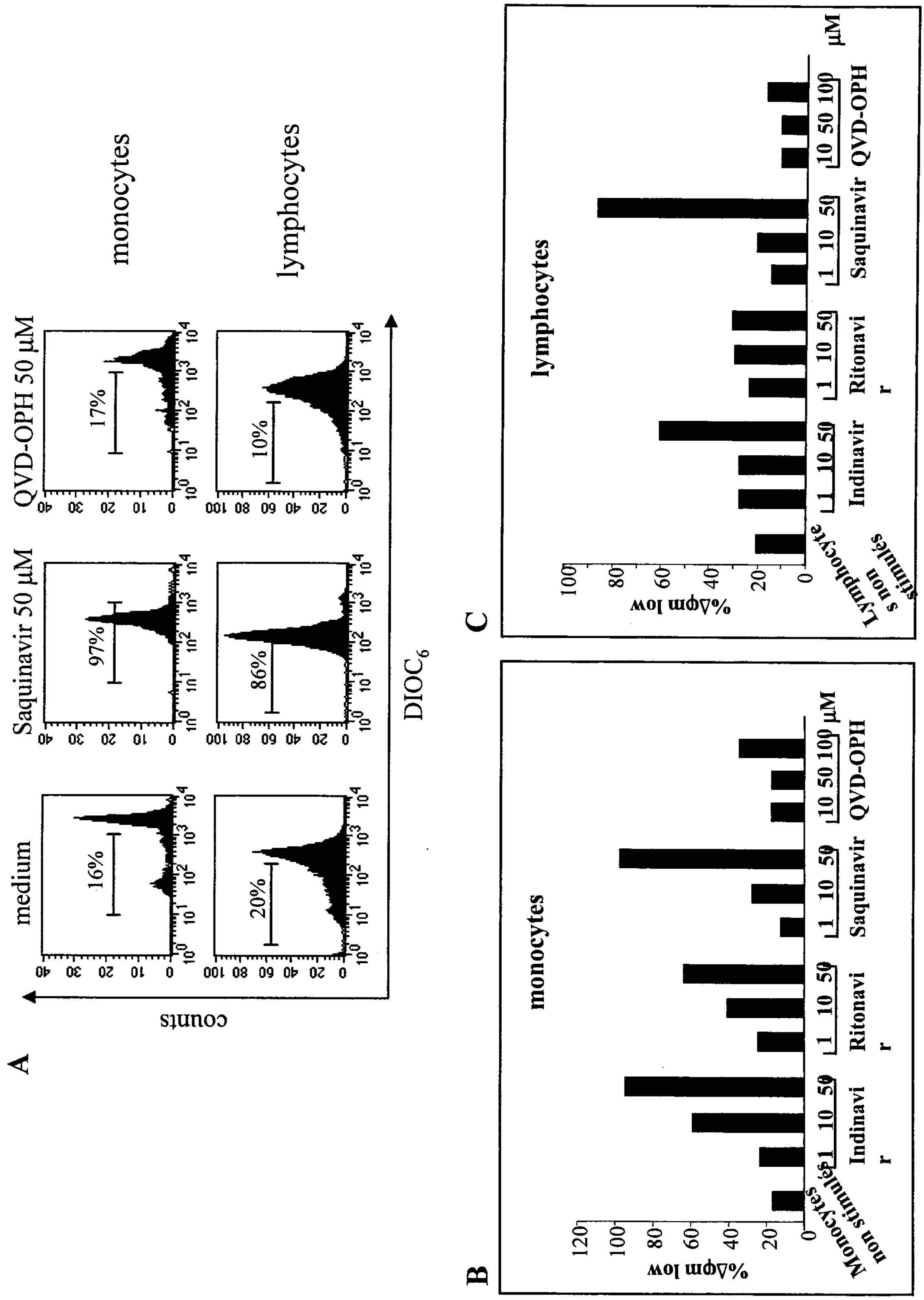
A
B
C
medium
Saquinavir 50 µM
QVD-OPH 50 µM
monocytes
lymphocytes
16%
97%
17%
20%
86%
10%
counts
DIOC6
%Δφm low
Monocytes non stimulés
Lymphocytes non stimulés
Indinavir
Ritonavir
Saquinavir
QVD-OPH
1 10 50
10 50 100
µM

Figure 15

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 02183341 A


### Littérature non-brevet citée dans la description


- Remington's Pharmaceutical Sciences. Mack Publishing Company
- **ALAM et al.** *J. Exp. Med.,* 1999, vol. 190 (12), 1879-1890
- **AMENDOLA et al.** *Proc Natl Acad Sci USA.,* 1996, vol. 93 (20), 11057-62
- **BARBER.** *Cell Death and Diff.,* 2001, vol. 8, 113-126
- **CASERTA et al.** *Apoptosis,* 2003, vol. 8 (4), 345-52
- **CHINNAIYAN et al.** *Nature Medicine,* 1997, vol. 3 (3), 333-337
- **CRYNS et al.** *Genes & Development,* 1998, vol. 12, 1551-1570
- **EVERETT ; MCFADDEN.** *Trends in Microbiology,* 1999, vol. 7, 160-165
- **FINKEL et al.** *Nat Med.,* 1995, vol. 1 (2), 129-34
- **GANDHI et al.** *The Journal of Experimental Medecine,* 1998, vol. 187 (7), 1113-1122
- The call of the wild: what can be learned from studies of SIV infection of natural hosts?. **GORDON et al.** HIV Sequence Compendium, 2005. Theoretical Biology and Biophysics Group. Los Alamos National Laboratory, 2005, 2-29
- **GOUGEON et al.** *J Immunol.,* 1996, vol. 156 (9), 3509-20
- **HURTREL et al.** *Cell Death Differ,* 2005, vol. 12, 979
- **LAVRIK et al.** *The journal of Clinical Investigation,* 2005, vol. 115 (10), 2665-2672
- **LEVINE et al.** *Proc Nanti Acad Sci.,* 1996, vol. 93, 4810
- **LIANG et al.** *J Virol.,* 1998, vol. 72, 8586
- **OLSEN et al.** *J Virol.,* 1996, vol. 70, 663
- **PETIT et al.** *J Biol Chem.,* 2002, vol. 277, 1477
- **STICHT et al.** *Nat Struct Mol Biol.,* 2005, vol. 12, 671
- **TERNOIS et al.** *Nat Struct Mol Biol.,* 2005, vol. 12, 678
- **THORNBERRY ; LABZEBNIK.** *Science,* 1998, vol. 281, 1312
- **VERA et al.** *Biology of Reproduction,* 2005, vol. 72, 516-522
- **WURZER et al.** *EMBO J.,* 2003, vol. 22, 2717